# EUROPEAN PATENT SPECIFICATION

(11) **EP 2 125 879 B1**
(45) Date of publication and mention of the grant of the patent: **07.09.2016**
(21) Application number: 08735412.2
(22) Date of filing: 27.02.2008
(51) Int. Cl.: C07K 14/435

(54) **IMMUNE SYSTEM MEDIATOR**
IMMUNSYSTEMMEDIATOR
MÉDIATEUR DU SYSTÈME IMMUNITAIRE

(30) Priority: 28.02.2007 GB 0703887
(43) Date of publication of application: 02.12.2009
(73) Proprietor: Arabian Gulf University, Manama (BH); Bakhiet, Abdelmoiz, Manama (BH)
(72) Inventor: BAKHIET, Abdelmoiz, P.O. Box 26671 Manama (BH); TAHA, Safa, P.O. Box 26671 Manama (BH)
(74) Representative: Cowley, Catherine Mary
(86) International application number: PCT/EP2008/052399
(87) International publication number: WO 2008/104575

(56) References cited:
- ELENKOV I J ET AL: "THE SYMPATHETIC NERVE-AN INTEGRATIVE INTERFACE BETWEEN TWO SUPERSYSTEMS: THE BRAIN AND THE IMMUNE SYSTEM" PHARMACOLOGICAL REVIEWS, WILLIAMS AND WILKINS INC., BALTIMORE, MD, US, vol. 52, no. 4, 1 December 2000 (2000-12-01), pages 595-638, XP001050579 ISSN: 0031-6997
- HUNT JOHN E ET AL: "Mouse mast cell protease 9, a novel member of the chromosome 14 family of serine proteases that is selectively expressed in uterine mast cells" JOURNAL OF BIOLOGICAL CHEMISTRY, vol. 272, no. 46, 14 November 1997 (1997-11-14), pages 29158-29166, XP002491097 ISSN: 0021-9258
- DATABASE Geneseq [Online] 4 December 2003 (2003-12-04), "Murine IRAK-M protein." XP002491098 retrieved from EBI accession no. GSP:ADB61346 Database accession no. ADB61346
- DATABASE EMBL [Online] 26 April 1999 (1999-04-26), "vt17f12.y1 Barstead mouse myotubes MPLRB5 Mus musculus cDNA clone IMAGE:1163375 5', mRNA sequence." XP002491099 retrieved from EBI accession no. EMBL:AI587713 Database accession no. AI587713
- DATABASE EMBL [Online] 04 June 2004 'Mus musculus molossinus DNA, clone:MSMg01-374I17.T7, genomic survey sequence.' Retrieved from EBI, accession no. EM_GSS:AG480315 Database accession no. AG480315
- MOIZ BAKHIET ET AL: "A novel nervous system-induced factor inducing immune responses in the spleen", IMMUNOLOGY AND CELL BIOLOGY, vol. 86, no. 8, 9 September 2008 (2008-09-09), pages 688-699, XP055052862, ISSN: 0818-9641, DOI: 10.1038/icb.2008.57

## Description

The present invention relates to an immune system mediator and particularly a novel polypeptide which is induced by a nervous stimulus and which has been found to mediate the transmission of signals between the immune system and the nervous system following an immune challenge.

A bewildering array of infectious agents and parasites gain subsistence at the expense of their hosts. Although host-parasite interplay depends on the virulence of parasites and resistance of the host, the early events of innate immunity during host-parasite interactions are very important in directing the ultimate pattern of the immune response. These early events of the innate immune response are, however, much less well characterized than the later secondary immune responses.

Immune responses are not isolated from other organ systems in the body, and indeed there are various means of communication between these organ systems and the immune system. Among these communications are regulatory interactions between the nervous system and the immune system. The nervous system of vertebrates is divided into the central nervous system (CNS), consisting of the brain and spinal cord, and the peripheral nervous system (PNS), consisting of all the other nerves that do not form part of the CNS. The PNS is further sub-divided into sympathetic, parasympathetic and enteric systems. The sympathetic nervous system prepares the body for stressful situations and activates what is often termed the 'fight or flight' response. This response is also known as the sympatho-adrenal response of the body, as adrenergic nerves release noradrenaline and adrenaline as neurotransmitters for the sympathetic nervous system. Channels of contact between the nervous system and the immune system include noradrenergic sympathetic innervation of the primary and secondary lymphoid organs in addition to stimulation of lymphocytes and other immuno-competent cells by virtue of their β- and α-adrenergic receptors (Hadden et al 1970, Sanders and Munsson 1985, Saito H 1990, Madden and Livnat 1991, Elenkov et al 2000).

The nervous-to-immune signalling pathway of early natural responses to infection has previously been investigated and the spleen has been shown to play a major role. The spleen, which is an important lymphoid organ involved in immune responses against all types of antigen that appear in the circulation, is richly innervated by adrenergic neurons that enter its parenchyma and remain largely associated with the splenic vasculature.

Recent studies have illustrated that cells within the central nervous system (CNS) have the capacity to produce molecules of the innate immunity, which during certain challenges can cause inflammatory and\or autoimmune diseases. Such molecules have been also demonstrated to act as important modulators of the fate of neurons (Simard and Rivest 2005) and were shown to be vitally involved in brain development (Bakhiet et al 2001).

Critical interactions between the nervous system and the immune system were further investigated during experimental autoimmune Myasthenia gravis (EAMG). Substantial effects of the nervous system on immune responses that influence the outcome of EAMG were shown, as surgical denervation of the spleen significantly reduced the clinical severity of the disease, suppressed the numbers of IgG and IFN-γ-secreting cells, down-regulated the mRNA expression for cytokines and reduced corticosterone and PGE₂ production (Bakhiet et al 2006). Also, immune responses to the parasite infection in the early stages of experimental African trypanosomiasis (EAT) were found to be modulated through autonomic innervation of the spleen (Liu et al 2000, Liu et al 2004), but the factor(s) that are involved in this mechanism are not known.

To date there has not been identified any single or group of molecules that function as mediators between the nervous and immune systems in response to a nervous stimulus following an immune challenge. The present inventors, have, however now identified such a molecule and which molecule may be particularly advantageous in further understanding the mechanism for innate immunity commencement and action. Furthermore, such a mediator also offers particular benefits as a potential therapeutic agent for modulating the natural responses in immune system disorders or in immunosupressed or immunocompromised individuals. In addition, compounds or molecules that inhibit that function of such a mediator may be particularly advantageous as a therapeutic treatment in individuals with an overstimulated immune system.

Therefore, in accordance with a first aspect of the present invention, there is provided an isolated nucleic acid molecule encoding a polypeptide designated herein as ISRAA (Immune System-Released Activating agent) which polypeptide has advantageously been found to play an important mediation role in transmission of signals between the nervous system and the immune system. The nucleic acid molecule of the present invention may comprise the full length nucleotide sequence for *israa* (SEQ ID No: 1) or consist of that of the open-reading frame identified in SEQ ID No: 2 or sequences complementary thereto. In another embodiment, the present invention provides nucleic acid molecules that code for a polypeptide having an amino acid sequence exhibiting any of at least 70%, 75%, 80%, 85%, 90%, 95% or 99% homology or identity to the amino acid sequence according to SEQ ID No: 3.

The term "isolated" refers to a nucleic acid substantially free of cellular material or culture medium when produced by recombinant DNA techniques, or chemical precursors or other chemicals when chemically synthesized. An "isolated" nucleic acid molecule is also free of sequences which naturally flank the nucleic acid (i.e., sequences located at the 5' and 3' ends of the nucleic acid) in the organism from which the nucleic acid is derived. The term "nucleic acid" molecule is intended to include DNA and RNA and can be either double stranded or single stranded. In one embodiment, the nucleic acid is a cDNA comprising a nucleotide sequence shown in SEQ ID NO: 1. In another embodiment, the nucleic acid is a cDNA consisting of the coding region of the nucleotide sequence, shown in SEQ ID NO: 2. In another embodiment, the nucleic acid encodes a protein comprising an amino acid sequence shown in SEQ ID NO: 3.

There are provided nucleic acids having substantial sequence homology with the nucleotide sequence shown in SEQ ID NO: 1 or SEQ ID NO 2 or encoding proteins having substantial homology to the amino acid sequence shown in SEQ ID NO: 3. Homology refers to sequence similarity between sequences and can be determined by comparing a position in each sequence which may be aligned for purposes of comparison. When a position in the compared sequence is occupied by the same nucleotide base or amino acid, then the molecules are homologous at that position. A degree of homology between sequences is a function of the number of matching or homologous positions shared by the sequences.

The term "nucleic acid sequences having substantial sequence homology" means those nucleotide sequences which have slight or inconsequential sequence variations from the sequences disclosed in SEQ ID No: 1 and SEQ ID No: 2, i.e. the homologous nucleic acids function in substantially the same manner to produce substantially the same polypeptides as the actual sequences. Alternative splice variants corresponding to a cDNA of the invention are also encompassed.

"Percent (%) amino acid sequence identity" with respect to the ISRAA polypeptide sequences identified herein is defined as the percentage of amino acid residues in a candidate sequence that are identical with the amino acid residues in the specific ISRAA polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared.

Percent amino acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span=1, overlap fraction=0.125, word threshold (T)=11, and scoring matrix=BLOSUM62. When WU-BLAST-2 is employed, a % amino acid sequence identity value is determined by dividing (a) the number of matching identical amino acid residues between the amino acid sequence of the ISRAA polypeptide of interest having a sequence derived from the native ISRAA polypeptide and the comparison amino acid sequence of interest (i.e., the sequence against which the ISRAA polypeptide of interest is being compared which may be a ISRAA variant polypeptide) as determined by WU-BLAST-2 by (b) the total number of amino acid residues of the ISRAA polypeptide of interest. For example, in the statement "a polypeptide comprising an the amino acid sequence A which has or having at least 80% amino acid sequence identity to the amino acid sequence B", the amino acid sequence A is the comparison amino acid sequence of interest and the amino acid sequence B is the amino acid sequence of the ISRAA polypeptide of interest.

Percent amino acid sequence identity may also be determined using the sequence comparison program NCBI-BLAST2 (Altschul et al., Nucleic Acids Res. 25:3389-3402 (1997)). NCBI-BLAST2 uses several search parameters, wherein all of those search parameters are set to default values including, for example, unmask=yes, strand=all, expected occurrences=10, minimum low complexity length=15/5, multi-pass e-value=0.01, constant for multi-pass=25, dropoff for final gapped alignment=25 and scoring matrix=BLOSUM62

There is provided a nucleic acid which hybridizes under high
or low stringency conditions to a nucleic acid having all or a portion of a nucleic acid sequences shown in SEQ ID No:1 or 2 or a nucleic acid encoding the amino acid sequence of SEQ ID No :3.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

Isolated nucleic acids encoding the polypeptide of the invention, and having a sequence which differs from a nucleotide sequence shown in SEQ ID NO: 1 or 2 due to degeneracy in the genetic code are also provided. Such
nucleic acids encode functionally equivalent proteins (e.g., a protein induced by the first nervous-to-immune signal) but differ in sequence from the sequence of SEQ ID NO: 1 or 2 due to degeneracy in the genetic code. Degeneracy means that a number of amino acids are designated by more than one triplet. DNA sequence polymorphisms that do lead to changes in the amino acid sequences of an ISRAA protein will also exist within a population. Any and all such nucleotide variations and resulting amino acid polymorphisms are provided.

The present invention also relates to an antisense nucleic acid, or oligonucleotide fragment thereof, of a nucleic acid of the invention. An antisense nucleic acid can comprise a nucleotide sequence which is complementary to a coding strand of a nucleic acid, e.g. complementary to an mRNA sequence, constructed according to the rules of Watson and Crick base pairing, and can hydrogen bond to the coding strand of the nucleic acid. The antisense sequence complementary to a sequence of an mRNA can be complementary to any sequence found in the coding region of the mRNA or can be complementary to a 5' or 3' untranslated region of the mRNA. Furthermore, an antisense nucleic acid can be complementary in sequence to a regulatory region of the gene encoding the mRNA, for instance a transcription initiation sequence or regulatory element. Preferably, an antisense nucleic acid complementary to a region preceding or spanning the initiation codon or in the 3' untranslated region of an mRNA is used. An antisense nucleic acid can be designed based upon the nucleotide sequences shown in SEQ ID NO: 1 or 2.

The present invention provides recombinant vectors comprising nucleic acid molecules that encode the polypeptide of the invention, as described herein. These recombinant vectors may be plasmids. In other embodiments, these recombinant vectors are prokaryotic or eukaryotic expression vectors. The nucleic acid coding for the polypeptides of the invention may also be operatively linked to a regulatory control sequence.

The nucleic acids of the present invention which encode the polypeptides of the invention can be incorporated into a recombinant expression vector using techniques known in the art, thus ensuring good expression of the encoded protein or part thereof. The recombinant expression vectors are "suitable for transformation of a host cell", which means that the recombinant expression vectors contain a nucleic acid or an oligonucleotide fragment thereof of the invention in addition to a regulatory sequence, selected on the basis of the host cells to be used for expression, which is operatively linked to the nucleic acid or oligonucleotide fragment. Operatively linked is intended to mean that the nucleic acid is linked to a regulatory sequence in a manner which allows expression of the nucleic acid. Therefore, nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice. Regulatory sequences are art-recognized and are selected to direct expression of the desired protein in an appropriate host cell. Accordingly, the term regulatory sequence includes promoters, enhancers and other expression control elements. Such regulatory sequences are known to those skilled in the art.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Expression of these recombinant expression vectors is carried out in prokaryotic or eukaryotic cells using standard molecular biology techniques.

The recombinant expression vectors of the invention can be used to make a transformant host cell including the recombinant expression vector. The term "transformant host cell" is intended to include prokaryotic and eukaryotic cells which have been transformed or transfected with a recombinant expression vector of the invention. The terms "transformed with", "transfected with", "transformation" and "transfection" are intended to encompass introduction of nucleic acid (e.g. a vector) into a cell by one of many possible techniques known in the art. Prokaryotic cells can be transformed with nucleic acid by, for example, electroporation or calcium-chloride mediated transformation. Nucleic acid can be introduced into mammalian cells via conventional techniques such as calcium phosphate or calcium chloride co-precipitation, DEAE-dextran-mediated transfection, lipofectin, electroporation, microinjection or any other known technique.

The present invention further provides host cells comprising a nucleic acid that codes for the polypeptides of the invention. The invention also provides a method of preparing the protein ISRAA. The method comprises culturing a transformant host cell including a recombinant expression vector comprising a nucleic acid of the invention and a regulatory sequence operatively linked to the nucleic acid in a suitable medium and thereafter isolating the protein.

Nucleic acids that encode the polypeptides of the invention can be used to generate either transgenic animals or "knock out" animals that, in turn, may be useful in further understanding the mechanism of action of ISRAA. A transgenic mammal (e.g. a rat or a mouse) is a mammal having cells that contain a transgene, which was introduced into the mammal or an ancestor of the mammal at a prenatal, e.g. an embryonic stage. A transgene is a DNA molecule which is integrated into the genome of a cell from which a transgenic animal develops.

The nucleic acid molecules that code for mammalian ISRAA proteins, such as mouse ISRAA, can be contained within recombinant vectors such as plasmids, phages, viruses, transposons, cosmids or artificial chromosomes. Such vectors can also include regulatory elements that control the replication and expression of the *israa* nucleic acid sequences. The vectors can also contain sequences that allow for the screening or selection of cells containing the vector. Such screening or selection sequences can include antibiotic resistance genes. The recombinant vectors can be prokaryotic expression vectors or eukaryotic expression vectors. The nucleic acid encoding *israa* can be linked to a heterologous promoter.

A nucleic acid molecule is a "polynucleotide" which is a single- or double-stranded polymer of deoxyribonucleotide or ribonucleotide bases read from the 5' to the 3' end. Polynucleotides include RNA and DNA, and may be isolated from natural sources, synthesized in vitro, or prepared from a combination of natural and synthetic molecules. Sizes of polynucleotides are expressed as base pairs (abbreviated "bp"), nucleotides ("nt"), or kilobases ("kb"). Where the context allows, the latter two terms may describe polynucleotides that are single-stranded or double-stranded. When the term is applied to double-stranded molecules it is used to denote overall length and will be understood to be equivalent to the term "base pairs". It will be recognized by those skilled in the art that the two strands of a double-stranded polynucleotide may differ slightly in length and that the ends thereof may be staggered as a result of enzymatic cleavage; thus all nucleotides within a double-stranded polynucleotide molecule may not be paired.

"Probes and/or primers" as used herein can be RNA or DNA. DNA can be either cDNA or genomic DNA. Polynucleotide probes and primers are single or double-stranded DNA or RNA, generally synthetic oligonucleotides, but may be generated from cloned cDNA or genomic sequences or its complements. Analytical probes will generally be at least 20 nucleotides in length, although somewhat shorter probes (14-17 nucleotides) can be used. PCR primers are at least 5 nucleotides in length, preferably 15 or more nt, more preferably 20-30 nt. Short polynucleotides can be used when a small region of the gene is targeted for analysis. For gross analysis of genes, a polynucleotide probe may comprise an entire exon or more. Probes can be labeled to provide a detectable signal, such as with an enzyme, biotin, a radionuclide, fluorophore, chemiluminescer, paramagnetic particle and the like, which are commercially available from many sources, such as Molecular Probes, Inc., Eugene, OR, and Amersham Corp., Arlington Heights, IL, using techniques that are well known in the art.

The present invention further provides an isolated polypeptide, designated herein as ISRAA. In a preferred embodiment, the protein has an amino acid sequence that has exhibits at least 70%, 75%, 80%, 85%, 90%, 95% or 99% homology or identity to the amino acid sequence according to SEQ ID No: 3.

A "polypeptide" is a polymer of amino acid residues joined by peptide bonds, whether produced naturally or synthetically. Polypeptides of less than about 10 amino acid residues are commonly referred to as "peptides". A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

Essential amino acids in the polypeptides of the present invention can be identified according to procedures known in the art, such as site-directed mutagenesis or alanine-scanning mutagenesis (Cunningham and Wells, Science 244: 1081-5, 1989 ; Bass et al., Proc. Natl. Acad. Sci. USA 88:4498-502, 1991). In the latter technique, single alanine mutations are introduced at every residue in the molecule, and the resultant mutant molecules are tested for biological activity (e.g. ligand binding and signal transduction) as disclosed below to identify amino acid residues that are critical to the activity of the molecule. See also, Hilton et al., J. Biol. Chem. 271:4699-4708, 1996. Sites of ligand-receptor, protein-protein or other biological interaction can also be determined by physical analysis of structure, as determined by such techniques as nuclear magnetic resonance, crystallography, electron diffraction or photoaffinity labeling, in conjunction with mutation of putative contact site amino acids. See, for example, de Vos et al., Science 255:306-312, 1992 ; Smith et al., J. Mol. Biol. 224:899-904, 1992 ; Wlodaver et al., FEBS Lett. 309:59-64, 1992. The identities of essential amino acids can also be inferred from analysis of homologies with related receptors.

Determination of amino acid residues that are within regions or domains that are critical to maintaining structural integrity can be determined. Within these regions one can determine specific residues that will be more or less tolerant of change and maintain the overall tertiary structure of the molecule. Methods for analyzing sequence structure include, but are not limited to, alignment of multiple sequences with high amino acid or nucleotide identity and computer analysis using available software (e.g., the Insight II viewer and homology modeling tools; MSI, San Diego, CA), secondary structure propensities, binary patterns, complementary packing and buried polar interactions (Barton, Current Opin. Struct. Biol. 5:372-376, 1995 ; and, Cordes et al., Current Opin. Struct. Biol. 6:3-10, 1996). In general, when designing modifications to molecules or identifying specific fragments determination of structure will be accompanied by evaluating activity of modified molecules.

The present invention also relates to a method for preparing isolated polypeptides according to the invention which method comprises culturing a transformant host cell including a recombinant expression vector in a suitable medium until said polypeptide is formed, and subsequently isolating the polypeptide. The steps in such a method represent standard laboratory techniques.

Host cells comprising a nucleic acid that codes for mammalian ISRAA are also provided. Host cells comprising a nucleic acid that is operatively linked to the regulatory sequence of the recombinant expression vector to allow expression of an RNA molecule which is antisense to the isolated nucleic acid of SEQ ID No: 1 or 2 are also provided. The host cells can be prepared by
transfecting an appropriate nucleic acid into a cell using transfection techniques known in the art. These techniques include calcium phosphate co-precipitation, microinjection, electroporation and liposome-mediated gene transfer.

The present invention further provides an antibody or antigen-binding fragment specific for an epitope of a protein of the invention. The antibodies or antigen-binding fragments may be polyclonal or monoclonal. Such polyclonal or monoclonal antibodies or antigen-binding fragments may be coupled to a detectable substance. The antibodies can be incorporated in compositions suitable for administration in a pharmaceutically acceptable carrier.

Immunogenic portions of the protein of the invention can be used to prepare antibodies specific for the proteins. Antibodies can be prepared which bind to an epitope in a region of the protein. The term antibody is also intended to include fragments which are specifically reactive with a protein, or peptide thereof, of ISRAA. Antibodies can be fragmented using conventional techniques, for example, F(ab')2 fragments can be generated by treating antibody with pepsin. The resulting F(ab')2 fragment can be treated to reduce disulfide bridges to produce Fab' fragments. Polyclonal antibodies are antibodies that are derived from different B-cell lines and are a mixture of immunoglobulin molecules secreted against a specific antigen, each recognising a different epitope. Monoclonal antibodies are antibodies that are identical because they were produced by one type of B-cell and are all clones of a single parent cell. Standard techniques are used to produce polyclonal antibodies. Routine procedure based on the hybridoma technique originally developed by Kohler and Milstein (Nature 256,495497 (1975)) is used to produce monoclonal antibodies.

"Antibody fragments" comprise a portion of an intact antibody, preferably the antigen binding or variable region of the intact antibody. Examples of antibody fragments include Fab, Fab', F(ab')2, and Fv fragments; diabodies; linear antibodies (Zapata et al., Protein Eng. 8(10):1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')2 fragment that has two antigen-combining sites and is still capable of cross-linking antigen.

"Fv" is the minimum antibody fragment which contains a complete antigen-recognition and -binding site. This region consists of a dimer of one heavy-and one light-chain variable domain in tight, non-covalent association. It is in this configuration that the three CDRs of each variable domain interact to define an antigen-binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2.

"Single-chain Fv" or "sFv" antibody fragments comprise the VH and VL domains of antibody, wherein these domains are present in a single polypeptide chain. Preferably, the Fv polypeptide further comprises a polypeptide linker between the VH and VL domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

The antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and coworkers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of ahumanantibody. Accordingly, such "humanized" antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).

A pharmaceutical composition suitable for administration comprising a polyclonal antibody or a monoclonal antibody is also within the scope of the invention. An antibody composition can be administered to a subject in an appropriate carrier or diluent, co-administered with enzyme inhibitors or in an appropriate carrier such as liposomes. The term "pharmaceutically acceptable carrier" as used herein is intended to include diluents such as saline and aqueous buffer solutions.

The present invention also provides a method for stimulating the innate immune response *in vitro* in immuno-suppressed cells and a method for inhibiting the innate immune response *in vitro* in certain cells. Therapeutic kits for performing such methods are also provided.

Therefore, in a further aspect the invention provides a method for stimulating the innate immune response *in vitro* in immuno-suppressed cells comprising contacting the cells with a purified polypeptide according to the invention.

In a further embodiment, the invention comprises a method for stimulating the innate immune response *in vitro* in immuno-suppressed cells comprising transfecting the cells with an isolated nucleic acid molecule according to the invention using a recombinant expression vector as described herein.

Also provided by the present invention is a method of identifying compounds or agents that inhibit or stimulate activity or expression of the polypeptide according to the invention and which method comprises contacting *in vitro* a cell expressing said polypeptide with a candidate compound or agent and establishing whether in response to an immune challenge the compound or agent inhibits or enhances the immune response when compared to a cell that has not been contacted with said compound.

The present invention further provides pharmaceutical compositions suitable for administration comprising such compounds or agents discussed herein, together with a pharmaceutically acceptable carrier.

The present invention also provides non-human transgenic mammals which contain cells transfected with a nucleic acid according to SEQ ID No: 1 or 2. There are provided non-human transgenic mammals which
contain cells having a gene that normally comprises a nucleotide sequence having substantial sequence homology to the nucleic acid of SEQ ID No:1 or 2, wherein said gene has been altered by homologous recombination such that the animal cannot express functional ISRAA protein.

There are provided non-human transgenic mammals which contain
cells transfected with a nucleic acid wherein the nucleic acid comprises a nucleotide sequence or complementary nucleotide sequence thereof which is capable of hybridising under at least low stringency conditions to the isolated nucleic acid of SEQ ID No: 1 or 2 or its complementary nucleotide sequence.

There are provided non-human transgenic mammals wherein one or
both alleles of the endogenous *israa* gene have been mutated. The non-human transgenic mammal can be a rat or a mouse or any suitable mammal.

The present invention also encompasses methods for modulating the immune system responses. In particular, the present invention includes a method for stimulating the innate immune response *in vitro* in immuno-suppressed cells using the nucleic acids or polypeptides of the invention. Such a method may comprise contacting cells with purified polypeptides of the invention and/or transfecting cells with isolated nucleic acids according to the invention.

The present invention also provides a method for inhibiting the innate immune response in cells *in vitro* by inhibiting the expression or activity of ISRAA. Such a method of inhibiting the expression or activity of ISRAA may comprise contacting cells with a molecule which binds to a polypeptide according to the present invention. Preferably this molecule may be either a polyclonal antibody or a monoclonal antibody. Such a method of inhibiting the expression or activity of ISRAA may also comprise introducing into cells an isolated nucleic acid molecule or oligonucleotide capable of hybridising to a genetic sequence which encodes said ISRAA. This isolated nucleic acid molecule or oligonucleotide may be RNA, and may be complementary to the sense strand of a genetic sequence which encodes said ISRAA. Such isolated nucleic acid molecule or oligonucleotide may also be complementary in sequence to the following; a regulatory region of the gene which encodes ISRAA, a transcription initiation region of a gene which encodes ISRAA, a 5' or 3' untranslated region of a gene which encodes the ISRAA, or the translation initiation codon of a gene which encodes ISRAA.

A wide variety of test agents may be tested in the screening methods of the present invention. For example, small molecule compounds, known in the art, chemicals, nucleic acids, peptides and proteins such as hormones, antibodies, and portions thereof, may act as test agents. In one non-limiting example, the three- dimensional structure of the active site of ISRAA may be determined by crystallizing the complex formed by the protein and a known inhibitor. Rational drug design may then be used to identify new test agents by making alterations in the structure of a known inhibitor or by designing small molecule compounds that bind to the active site of the protein.

In another embodiment, RNA interference may be used as an inhibitor of ISRAA. RNA interference relates to sequence-specific, posttranscriptional gene silencing brought about by double-stranded RNA that is homologous to the silenced gene target. Methods for inhibiting production of a protein utilizing small interfering RNAs are well known to the art, and disclosed in, for example, PCT International Application Numbers WO01/75164 ; WO00/63364; WO01/92513 ; WO00/44895 ; and WO99/32619.

The agents may be administered by a wide variety of routes. Exemplary routes of administration include oral, parenteral, transdermal, and pulmonary administration. For example, the agents may be administered intranasally, intramuscularly, subcutaneously, intraperitonealy, intravaginally and any combination thereof. For pulmonary administration nebulizers, inhalers or aerosol dispensers may be used to deliver the therapeutic agent in an appropriate formulation (i. e., with anaerolizing agent). In addition, the agents may be administered alone or in combination with other agents or known drugs. In combination, agents may be administered simultaneously or each agent may be administered at different times. When combined with one or more known drugs, agents and drugs may be administered simultaneously or the agent can be administered before or after the drug(s).

In one embodiment, the agents are administered in a pharmaceutically acceptable carrier. Any suitable carrier known in the art may be used. Carriers that efficiently solubilise the agents are preferred. Carriers include, but are not limited to a solid, liquid or a mixture of a solid and a liquid. The carriers may take the form of capsules, tablets, pills, powders, lozenges, suspensions, emulsions or syrups. The carriers may include substances that act as flavoring agents, lubricants, solubilizers, suspending agents, binders, stabilizers, tablet disintegrating agents and encapsulating materials.

Tablets for systemic oral administration may include excipients, as known in the art, such as calcium carbonate, sodium carbonate, sugars (e. g., lactose, sucrose, mannitol, sorbitol), celluloses (e. g., methyl cellulose, sodium carboxymethyl cellulose), gums (e. g., arabic, tragacanth), together with disintegrating agents, such as maize, starch or alginic acid, binding agents, such as gelatin, collagen or acacia and lubricating agents, such as magnesium stearate, stearic acid or talc.

In powders, the carrier may be a finely divided solid, which is mixed with an effective amount of a finely divided agent. In solutions, suspensions or syrups, an effective amount of the agent may be dissolved or suspended in a carrier such as sterile water or an organic solvent, such as aqueous propylene glycol. Other compositions can be made by dispersing the inhibitor in an aqueous starch or sodium carboxymethyl cellulose solution or a suitable oil known to the art.

A therapeutic kit is included within the scope of the current invention for performing such methods to modulate the innate immune system responses. Preferably, these kits include means of adding the above-described molecules or agents to cells.

The present invention will be further described with reference to the following examples that illustrate the embodiments of the invention with further reference to the following drawings.

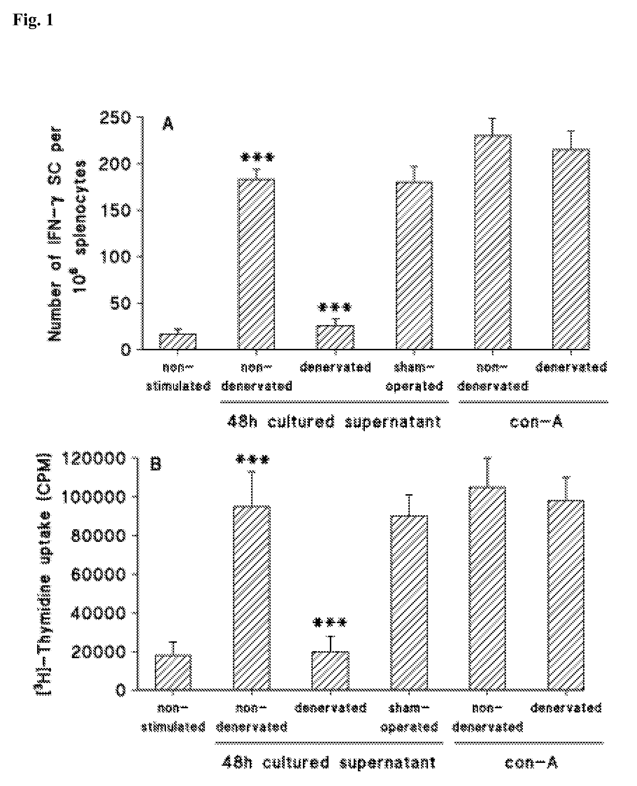

Figure 1 is a graphic representation illustrating action of ISRAA on naive cells to which splenocyte supernatants from trypanosome-inoculated rats have been added and the effects of splenic denervation on induction of ISRAA. (A) Splenocyte supernatants from *T.b.brucei* inoculated rats for less than one minute and cultured for 48h significantly stimulated naive cells to produce IFN-γ (p<0.0001) as detected by ELISPOT assay. Note: significant inhibition of the supernatant activity by splenic denervation (p<0.0001). Con-A was used as +ve control. Bars denote mean ± S.D (n=9). The experiments were repeated 6 times with similar results. (B) Splenocyte supernatants from *T*.*b*.*brucei* inoculated rats for less than one minute and cultured for 48h significantly stimulated naive cells to proliferate (p<0.0001) as detected by cell proliferation assay. The ³H-Thymidine uptake expressed in counts per minute (CPM) and was highly significant in the *T*.*b*.*brucei* inoculated non-denervated compared to PBS-inoculated non-denervated rats (p<0.0001). Sham operated-rats demonstrated high proliferative response non-denervated. Note: significant inhibition of the supernatant activity by splenic denervation (p<0.0001). Con-A was used as +ve control. Bars denote mean ± S.D (n=9). The experiments were repeated 6 times with similar results.

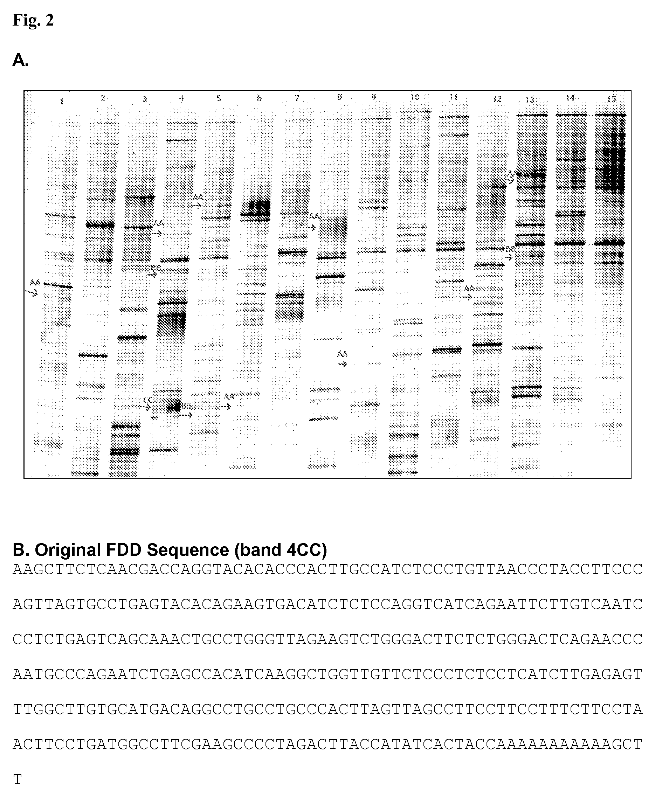

Figure 2A is an illustration of the results of a FDD used to further clone the gene for ISRAA .The FDD sheet show results of the automated FDD comprehensive screening using Horizontal FDD Electrophoresis System. Analysis of the cDNA bands and BDD and confirmation of differentially expressed genes revealed discovery of a new gene that is differentially expressed in splenocytes obtained in less than one minute from mice inoculated subcutaneously with *T*.*b*.*brucei*, but not in PBS-inoculated control mice (band 4CC).

Figure 2B illustrates the original sequence from the band 4CC from the FDD. GenBank searches identified no nucleotide sequence similarities.

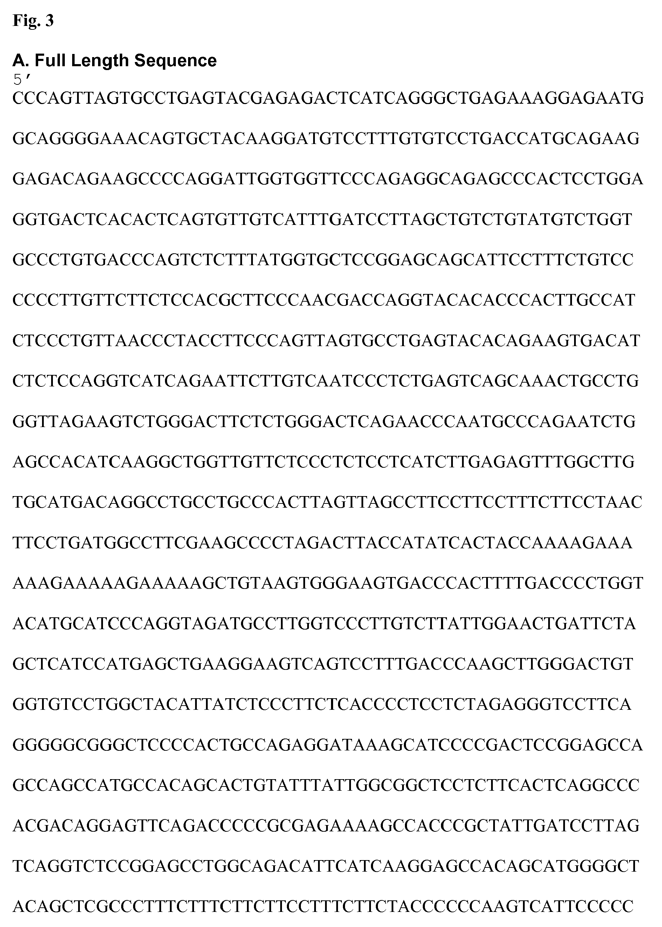

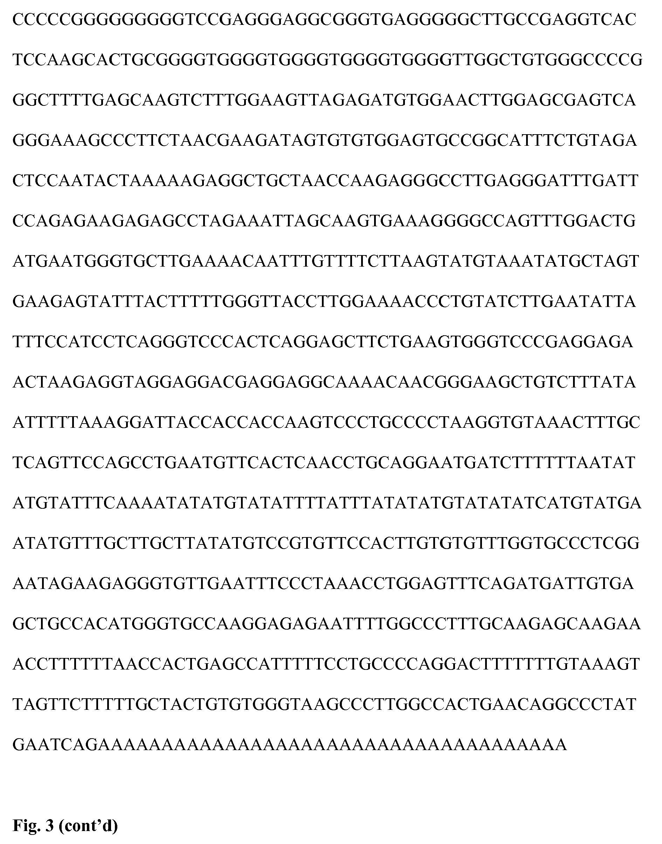

Figure 3A is the novel sequence of the *israa* gene of the present invention. To obtain the full length sequence of the cDNA for ISRAA, full length mRNA was cloned into a mammalian expression vector and the library was screened by specific primers constructed from the original FDD Sequence of band 4CC showed in Fig. 2B. The figure demonstrates full length sequence of the cDNA which showed 2093 residue sequence starting "CCCCCAGTTA". A GenBank search did not reveal any significant homologies. Protein expression was done and rISRAA was purified, characterized and tested for biological activities (see below).

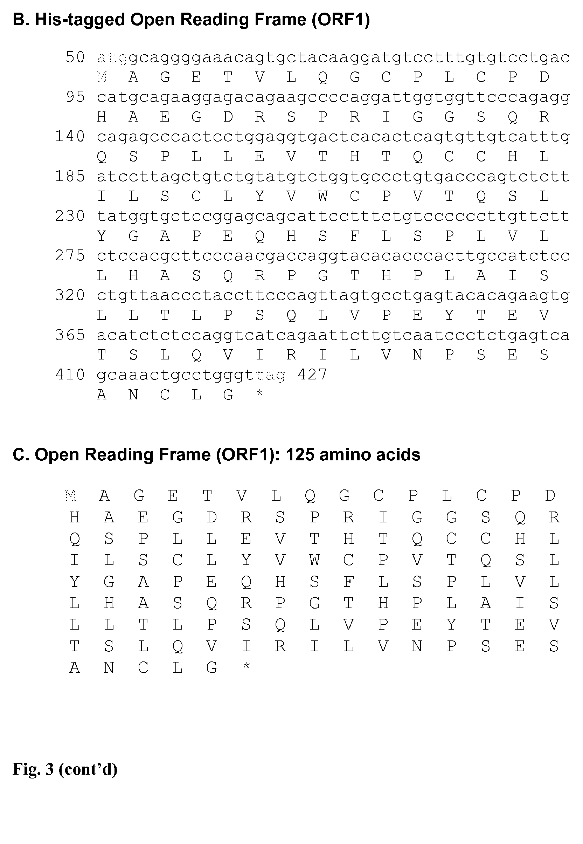

Figure 3B is the sequence open reading frame for the novel gene (His-tagged ORF)of the invention.

Figure 3C is the amino acid sequence of the open reading frame for the novel gene of the invention.

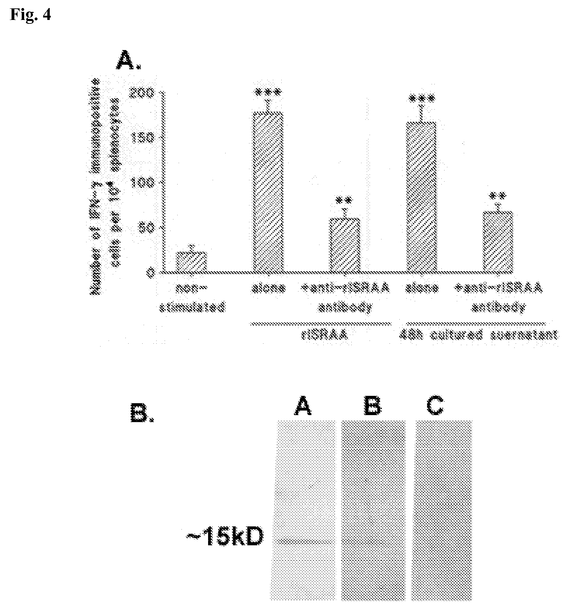

Figure 4A is a graphic representation illustrating actions of the cloned rISRAA and the splenocyte supernatants from trypanosome-inoculated rats on naive cells together with the blocking effects of the anti-ISRAA antibody on the activity. (A) rISRAA and splenocyte supernatants from *T.b.brucei* inoculated rats for less than one minute and cultured for 48h significantly stimulated naive cells to produce IFN-γ (p<0.0001) as detected by ELISPOT assay. Note: significant inhibition of rISRAA and the supernatant activities by pre-incubation of the cells with anti-ISRAA polyclonal antibody (p<0.001). Con-A was used as +ve control. Bars denote mean ± S.D (n=9). The experiments were repeated 6 times with similar results.

Figure 4B illustrates recognition of rISRAA and native ISRAA on splenocyte supernatants from trypanosome-inoculated rats by the anti-rISRAA antibody as detected by Western blot. SDS electrophoresis and Western blot using polyclonal rabbit anti-rISRAA antibody against rISRAA.
(A) rISRAA
(B) Splenocyte supernatant from trypanosome-inoculated rats collected from 48h cultured cells
(C) Control (supernatant collected from 48h cultured splenocytes prepared from subcutaneous PBS inoculated rats.
Note ∼15 kD bands in (A) and (B).

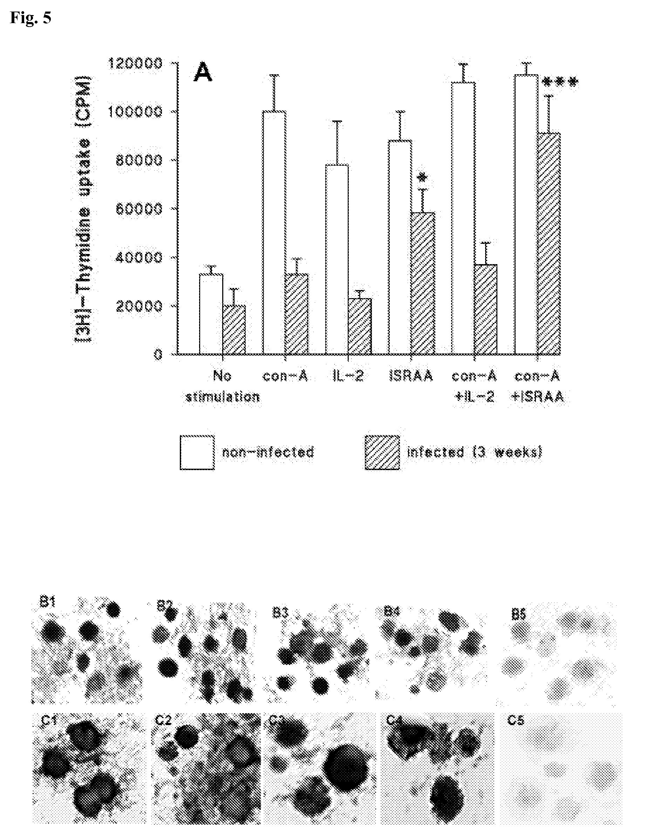

Figure 5A is a graphic representation illustrating the role of ISRAA in the modulation of disease immunosuppression .This graph demonstrates effects of ISRAA in immunosuppressed splenocytes. Cells obtained from non-denervated rats 3 weeks post infection failed to proliferate after con-A-stimulation. ISRAA was added either alone or to con-A-stimulated splenocytes obtained at week 3 after infection. Significant proliferative responses were registered (*=p<0.01, ***=p<0.0001). IL-2 was similarly incubated in control wells, but no increased proliferation was noted. The data represent pools of two experiments (Mean ± SD are shown).

Figure 5B illustrates cell types producing ISRAA. In these experiments, mice were subcutaneously inoculated for less than one minute with *T*.*b*.*brucei*. Splenocytes were isolated and enriched to obtain purified populations of (A) CD4⁺, (B) CD8⁺, (C) B cells and (D) monocytes/macrophages. Using immunohistochemistry all purified cells showed ability to produce ISRAA. (E) represents control experiments (splenocytes from non-inoculated mice).

Figure 5C illustrates types of cells responding to ISRAA. Mouse splenocytes were stimulated by rISRAA and thereafter CD4⁺ (A), CD8⁺ (B), B cells (C) and monocytes/macrophages (D) were purified and tested for IFN-γ production by immunohistochemistry. Note: All types of cells were positively stained for IFN-γ in response to ISRAA stimulation. (E) represents non-stimulated cells.

The results obtained provide the basis for the therapeutic potential of the novel ISRAA protein encoded by the novel *israa* gene. The experiments carried out to achieve these results are outlined in the following paragraphs.

The present work relates to a novel immune system mediator which has been found to mediate the transmission of signals between the immune system and the nervous system following an immune challenge. Supernatants of 48h cultured splenocytes prepared from subcutaneously trypanosome-inoculated rats spleens obtained directly after inoculation and added to naive cells showed increased IFN-γ production and cell proliferation, an action which was significantly blocked by surgical denervation of the spleen. To identify differentially expressed genes in this process the Florescent Differential Display technique was used. A new gene in mouse splenocyte was identified and sequenced. The full length mRNA was cloned into a mammalian expression vector and the library was screened by specific primers and full-length sequence of a novel gene was obtained and found to be located in chromosome 14. Protein expression was done and the recombinant protein was purified. Western blot analysis using rabbit polyclonal antibody against the protein demonstrated ∼15 kD molecular mass band. The recombinant protein showed similar biological activities as the cultured supernatants. Furthermore, the protein was able to reactivate experimentally immunosuppressed cells by regaining their ability to proliferate. Thus, sympathetic outflow was shown to be directed to the spleen in response to a trypanosma stimulus leading to genaration of a novel immune system released activating agent (ISRAA) within few seconds after the parasitic challenge.

African trypanosomiasis, a disease of tropical Africa affecting mainly the CNS and characterised by profound induction of cytokines such as IFN-γ early during infections (Olsson et al 1991, Olsson et al 1993, Vaidya et al 1997) and late immunosuppression (Askonas 1984, Wei G, Tabel H 2008), was used in this study as a model to demonstrate critical interactions between the CNS and the immune system. Within few seconds after parasitic challenge in EAT, a dramatic expression of a novel gene in the spleen together with uppregualtion of its encoding protein (Immune System-Relaesed Activating Agent; ISRAA) was depicted. The autonomic nervous system is critically involved in this process.

### Materials and Methods

### Animals, parasite strain and infection procedure

Sprague-Dawley rats (Alab, Stockholm) aged 2 months and weighing about 200 g was used. The *Trypanosma brucei brucei* (*T*.*b*.*brucei*) strain variable antigen type AnTat 1.1E isolated from the bushbuck was obtained from Dr. Nestor van Meirvenne, Laboratory of Serology - Institute of Tropical Medicine Prins Leopold - Antwerp - Belgium. Each rat (denervated or non-denervated sham-operated) was injected subcutaneously with 0.1 ml of a suspension of trypanosomes in a phosphate saline/glucose buffer, pH 8.0, containing about 10⁶ parasites per ml. Ethical approval was obtained from Stockholm South Committee for Animal Ethics - Sweden. All experiments were performed in accordance with relevant guidelines and regulations. To clone ISRAA gene and test its gene product (recombinant ISRAA; rISRAA), Balb/c mice strain aged 2 months and weighing about 25 g (supplied by the Animal Breeding Facility at the Faculty of Medicine of Arabian Gulf University - Kingdom of Bahrain) were used. The animals were housed 4 per cage, given food and water ad libidum, and maintained on a 12/12-h light/dark cycle until sacrifice.

### Surgical sympathectomy and sham operation

The rats were weighed before surgery. Under pentobarbital sodium anesthesia (50 mg/kg), the fur on the operating area was shaved and the skin was sterilized with 70 % alcohol. A five mm long incision was made through the skin and muscle layers to expose the abdominal cavity. The ligamentum gastrosplenicum was cut. The splenic nerve was isolated from the splenic vasculature and connective tissue near the bifurcation of the celiac artery, and then the entire bundle of the nerve was cut. Immediately after surgery, each animal received an intraperitoneal injection of sulfadimethoxide (100 mg/rat) and then returned to the colony. Successful denervation was confirmed by histological studies at the end of the study. Sham-operated control rats were manipulated similarly, but without sympathectomy. The animals were allowed to recover from surgery for 5 days, and one week later they were inoculated with the parasite.

### Preparation of splenocytes

10⁵ parasites were injected subcutaneously into (i) non-denervated (ii) denervated (iii) sham-operated rats (3 per group). Also, PBS-inoculated non-denervated rats were used as negative control (non-stimulated). Immediately after the injection, spleens were dissected and splenocytes were prepared by forcing the spleens through a stainless steel mesh. The freed cells were washed once in Dulbecco MOD Eagle Medium (Life Technology) supplemented with 2 mM L-glutamine (Flow), 50 IU penicillin (Astra, södertälje, Sweden), 1% (v/v) MEM (Flow) and 5% (v/v) FCS (Gibco, Paisley, Scotland). Erythrocytes in the cell pellets were haemolysed by adding 2 ml cold water for 30 sec followed by the addition of 1 ml 2.7% saline. The cells were washed twice in the wash medium and re-diluted to obtain a concentration of 5x10⁶ ml. 200 µl aliquots were applied to individual wells of a microtitre plate. No changes in the viability of the cells obtained from denervated or non-denervated rats were observed. Splenocytes from animals that were inoculated with parasites were cultured for 48 hours and thereafter supernatants were collected and added to naive splenocytes and tested for their ability to induce the naive cells to produce IFN-γ and to proliferate. The 48 h incubation period was selected after performing kinetic study to test the best activity in the screening assay.

### Cell enrichment

To obtain CD4⁺ and CD8⁺ T cells, B cells and monocyte/macrophages purified cell preparations mouse splenocyte suspensions were prepared as above. CD4⁺, CD8⁺ T cells, B cells and monocytes/macrophages purified cell populations were prepared using magnetic beads (Dynabeads) coated with anti-CD4, anti-CD8, anti-mouse IgG and anti-mouse PAN monocyte/macrophages monoclonal antibodies (mAbs), respectively, following the manufacturer's instruction (Dynal, Norway). Purified cell preparations were analyzed with fluorescent mAbs by Flow cytometry (Mix et al 1990).

### Single cell assay for IFN-γ production

To test activity in cultured supernatants and lately of rISRAA, the enzyme-linked immunospot (ELISPOT) assay was used to detect IFN-γ production by single secretory cells (Mustafa et al 1991). In principle, nitrocellulose-bottomed 96 well microtitre plates (Millipore, Bedford - MA) were coated overnight with 100 µl aliquots of the mouse monoclonal (mAb) DB1, which is specific for IFN-γ, at a concentration of 5 µg /ml (Van der Meide et al 1986). After repeated washings with PBS, 2% bovine serum albumin was applied for 2-4 h, the plates were washed in PBS, and splenocyte suspensions were applied followed by stimulation with splenocyte culture supernatants obtained from denervated and non-denervated rats inoculated with *T*.*b*.*brucei* or 5 µg of rISRAA. Some control cells were either stimulated with con-A, or kept without stimulation. After overnight incubation at 37°C in a humidified atmosphere of 7% CO₂, cells were removed by flicking the plate, followed by repeated washings in PBS. Polyclonal rabbit anti-rat IFN-γ (Van der Meide et al 1986), diluted 1/1000, was applied for 4h. After washing, biotinylated goat anti-rabbit IgG (Vector Lab, Burlingame, CA) was applied for 4 h followed by avidin-biotin-peroxidase complex (ABC Vectastain Elite Kit, Vector Lab). Color development with 3-amino-9-ethylcarbazole and H₂O₂ was performed. Spots corresponding to cells that had secreted IFN-γ were counted using a dissection microscope.

### ³H-Thymidine-incorporation assay

200 µl of splenocyte suspension (5×10⁶/ ml medium) were applied to each well of a round-bottom polystyrene 96-well microtitre plate (Nunclon, Nunc, Roskilde, Denmark). Six wells received either splenocyte culture supernatants obtained from denervated and non-denervated rats inoculated with *T*.*b*.*brucei*, or con-A at a final concentration of 5µg/ml or no stimulating agent. In certain experiments (see below) 5 µg of rISRAA or rIL-2 were added either alone or together with con-A to splenocytes obtained at late time points during the infection. The cells were incubated for 72h. Ten hours before harvest 10 µl aliquots containing 1 µCi of [³H]-methylthymidine (specific activity 42 Ci/mmol) (Amersham, Little Chalfont, UK) in saline were added to each well. Cells were harvested onto glass fiber filters with a multiple channel semiautomated harvesting device (Titertek, Skatron AS, Lierbyen, Norway) and thymidine incorporation was measured as counts per minute (CPM) in a liquid beta-scintillation counter (Mark II, Searle Analytic, Des Plaines, IL, US).

### Fluorescent Differential Display (FDD) assay

To isolate the gene for ISRAA the FDD assay was used (Liang, P. and A.B. Pardee 1992). Briefly, total cellular RNA was extracted from splenocyte cultures of two groups of animals (experimentally infected for few seconds, and non-infected PBS-inoculated controls) using RNApure™ Reagent (GenHunter Corporation, Nashville, TN) according to manufacturer. Message Clean® Kit (GenHunter Corporation, Nashville, TN) was used to remove any possible remaining traces of genomic DNA contaminants. Differential Display was performed as described in the instructions of the kit RNAspectra™ Green (Fluorescent mRNA Differential Display System-GenHunter ® Corporation, Nashville, TN). First strands cDNA (20µl) were synthesized for each RNA sample separately using one of three different one-base anchored H-T₁₁M primers (Where M may be G, A or C). 200ng total cellular RNA, 4µl 5X RT buffer, 1.6µl 4dNTPs (250µM) and 2µl T₁₁M primer (10pmol/µl) with H₂O to 19µl. The reaction tubes were incubated for 5 minutes at 65°C and for 10 minutes at 37°C. Then, 1µl MMLV reverse transcriptase (200U/µl) was added to all reaction tubes and incubation was continued for 50 minutes at 37°C. MMLV RT was inactivated by incubation for 5 minutes at 95°C. For each 20 µl PCR reactions, 9.2 µl H₂O were mixed with 2µl of 10X PCR buffer, 1.6 µl 4dNTP mix (25µM), 2µl arbitrary primer (H-AP primer 2µM), 2µl FH-T₁₁M primer (M may be G, A or C), 2µl cDNA and 0.2µl Taq DNA polymerase (5U/µl) (Qiagen). The PCR cycling profile was 40 cycles (94°C, 30sec.) (40°C, 2min.) (72°C, 60sec.), 1 cycle (72°C, 5min). The samples were kept in the dark after the PCR reaction.

Denatured FDDRT-PCR products were loaded onto a 6% denaturing polyacrylamide DNA sequencing gel (7M Urea, 1X TBE). 3.5µl of each sample was mixed with 2µl of FDD loading dye and incubated at 80°C for two minutes and electrophoresed for 2-3 hours at 60 watts constant power. The gel was scanned on a Fluorescence Imager (Hitachi FMBIO® II) with 505 nm filter following the manufacturer's instructions. Bands of interest were excised with a clean razor blade, soaked in 100µl H₂O for 10 min at room temperature (RT) in a microcentrifuge tube, then boiled for 10 minutes. Tubes were centrifuged and the supernatants containing DNA were transferred into fresh tubes and precipitated with 10µl of 3M NaAcetate, 5µl glycogen (10mg/ml), 400µl 100% ethanol by incubation at -70°C for at least 30minutes. Tubes were centrifuged for 10 minutes at 4°C to pellet the DNA which was then washed with 85% EtOH and the pellet was redissolved in 10 µl of dH₂O.

Re-amplification was done using the same primer combination and PCR conditions described earlier except that unlabeled H-T₁₁M anchor primer was used instead of Fluorescein labeled FH-T₁₁M. 40µl reaction mixture was prepared by mixing 23.3µl dH2O, 4µl of 10X PCR buffer, 0.3 µl dNTP mix, 4µl H-AP primer (2µM), 4µl H-T₁₁M, 4µl of the isolated cDNA and 0.4µl Taq DNA polymerase (Qiagen). 30µl of the PCR samples were run on 1.5% agarose gel and stained with ethidium bromide. The size of re-amplified PCR products was checked against their size on the original DNA sequencing gel. The isolated PCR fragment was used directly for cycle sequencing, sub-cloning or as probe for northern blots. Re-amplified bands were cloned into PCR-TRAP Cloning System (GenHunter® Corporation, Nashville, TN). Colonies for each band were checked for inserts by colony-PCR. DNA sequencing was performed on an Applied Biosystems (ABI-3100). Sequences were compared with National Centre of Biotechnology Information (NCBI) non-redundant sequence database using the BLASTX and BLASTN programs.

### Confirmation of ISRAA FDD gene expression

Binary Differential Display (BDD) method was used to confirm the expression of the ISRAA FDD expressed gene according to protocols of GenHunter Corporation, Nashville, TN, US). The same RNA samples from FDD screening of ISRAA target gene were analyzed by BDD.

### Cloning of ISRAA full length gene and protein expression and purification

The QIAexpressionist™ protocols were used for cloning of the full length ISRAA gene and for high-level expression and purification of 6xHis-tagged protein according to manufacturer instructions (QIAGEN Inc. Stanford, Valencia, CA, US). In brief, ISRAA full length gene was obtained by cloning the full length mRNA into a mammalian expression vector followed by screening of the library. Subcloning of the first ORF into bacterial expression vector as a His-Tag Fusion Protein was done followed by full-length cDNA analysis, ORF determination, reamplification of ORF using custom primers and PCR-cloning of ORF into PCR-TRAP cloning system, plasmid mini-prep and restriction enzyme digestion for ORF insert and bacterial expression vector PQE32. Gel purification of enzyme digestion for ORF insert and expression vector, subcloning ORF insert into expression vector to create His-tag fusion protein were then conducted and followed by sequencing to determine if correct ORF is added in-frame and to confirm His-tag was fused in-frame at N-terminus (x).

Protein expression and purification was done by inoculation of two constructs of bacterial expression vector in 5mL LB culture and induced protein expression with IPTG. One construct of bacterial expression vector was inoculated in 5mL LB culture followed by reinoculation of 1L LB culture with 5mL culture and induced protein expression. Column chromatography protein purification under denaturing conditions with Ni-NTA beads was then carried out followed by SDS-PAGE to determine if protein was expressed and purified and SDS-PAGE protein gel to determine if protein is expressed in supernatant.

### Anti-ISRAA polyclonal antibodies

A polyclonal antiserum was produced by immunizing 2 rabbits with gel purified ISRAA protein as a specific antigen. The antibody was checked for specificity by Western blot and by its biological activity to block ISRAA-induced IFN-γ production by splenocytes.

### Detection of ISRAA and IFN-γ by immunohistochemistry

In principle, immunostaining was performed as previously described (Sander et al 1991, Lore 1998). Briefly, cultured naive splenocytes were harvested after stimulation with splenocyte culture supernatants obtained from mice inoculated with *T.b.brucei.* The cells were washed in PBS and transferred to adhesion slides (BioRad Lab, Munich, Germany). Cells were allowed to adhere to the slides for 30 minutes at 37°C. Excessive cells were washed away. Cell fixation was performed in 2% formaldehyde in PBS at pH 7.4 for 10 minutes. The cells were then stored at -20°C until required for further investigation. Endogenous peroxidase was blocked with 1 % H₂O₂ in 1x Earl's balanced salt solution (BSS) (Gibco) supplemented with 0.01 M HEPES buffer (Gibco) and 0.1% saponin (Riedel-de Haen, Seelze, Germany) for 30 minutes at RT. In order to reduce risks for non-specific antibody and hydrophobic interactions, the following precautions were undertaken: incubation with 2% FBS for 5 minutes at 37°C followed by incubation with 1% normal mouse sera for 30 minutes at 37°C. Additional incubation with blocking kit (Vector Laboratories, Burlingame, CA) was performed to block endogenous biotin or biotin-binding proteins. Cells were permeabilized with 0.1% saponin dissolved in BSS to allow the intracellular access of the cytokine-specific antibody. Rabbit polyclonal anti-mouse IFN-γ (Van der Meide et al 1986) and rabbit anti-ISRAA polyclonal antibody diluted 1/000 diluted in BSS-saponin were added and allowed to incubate for 30 minutes at 37°C followed by several washes in BSS. Non-specific staining by the second-step biotinylated goat antibody caused by Fc-interactions was prevented by a subsequent incubation with 1% goat serum (Dako, Glostrup, Denmark) dissolved in BSS-saponin for 15 minutes at RT. After washing, biotinylated goat anti-rabbit IgG (Vector Lab, Burlingame, CA) was used at 1/600 dilution in BSS-saponin. After three additional BSS washes, the cells were incubated with an avidin-biotin horse-radish peroxidase complex (Vectastain, Vector Laboratories) for 30 minutes at RT. A colour reaction was developed by 3'-diaminobenzidine tetrahydrochloride (DAB) (Vector Laboratories) and stopped after 2-10 minutes in the dark by washes in BSS. The cells were counterstained with hematoxylin and the slides were left to dry before mounting in buffered glycerol. The immunocytochemically stained cells were examined in a Leica RXM microscope (Leica, Wetzlar, Germany) equipped with a 3CDD colour camera (Sony, Tokyo, Japan). Enumeration of cytokine producing cells was performed manually at X 630 original magnification. The frequency of cytokine expressing cells was assessed by examination of at least 10⁴ cells.

### SDS gel electrophoresis and Western blot

Collected supernatant of 48h cultured splenocytes prepared from mice spleens rapidly obtained after subcutaneous inoculation of *T*.*b*.*brucei* and rISRAA were analyzed by sodium dodecyl sulphate polyacrylamide gel electrophoresis (SDS-PAGE, Bio-Rad, CA, US). Gels were subjected to Western blot in principle according to Towbin et al 1979 using the rabbit polyclonal antiserum against rISRAA and alkaline phosphatase immunostaining. The substrates, NMT and BCIP, were dissolved in a 0.15 M veronal-acetate buffer, pH 9.6. Molecular weight standards were run in parallel.

### Statistical analysis

Mann-Whitney's test was used to calculate level of significance (*=p<0.01, **=p<0.001, ***=p<0.0001).

### Results

### Detection of ISRAA activity and effects of splenic denervation on ISRAA action

IFN-γ is a potential immunoregulatory cytokine of the innate and adaptive immune responses. Using IFN-γ immunoassays and ³H-Thymidine-incorporation assays, a hypothesis of essential nervous-immune communications in innate immunity was examined after collecting 48 hour splenocyte culture supernatants from rats challenged for less than one minute with subcutaneously inoculated *T*.*b*.*brucei*. These supernatants were then added to naive cells. Splenocyte supernatants from the trypanosome-inoculated rats significantly stimulated naïve cells to produce IFN-γ (Fig. 1A) and to proliferate (Fig. 1B) compared to non-stimulated PBS-inoculated cells (p<0001).

To examine whether the splenic nerve is directly involved in this activity, splenocyte supernatants collected from denervated rat spleens challenged subcutaneously with *T*.*b*.*brucei* for less than one minute and cultured for 48 hours did not show biological activity as the induction of IFN-γ and the proliferative responses were significantly reduced with splenic denervation (p<0001). Supernatants from sham-operated rats showed similar activity as those from non-denervated rats. Con-A was used as a positive control and showed high induction of IFN-γ and increased proliferation in both denervated and non-denervated splenocytes (Fig. 1).

### Fluorescent Differential Display (FDD) results

To identify genes that are differentially expressed in splenocytes obtained in less than one minute from mice inoculated subcutaneously with *T*.*b*.*brucei* compared to PBS-inoculated control mice the FDD technique was used to detect on/off and upregulated/downregulated genes. Results of the automated FDD comprehensive screening using Horizontal FDD Electrophoresis System are shown in Fig 2A. Twenty cDNA bands exhibited reproducible differences. All 20 cDNA bands were pursued for re-amplification, cloning and DNA sequencing. Analysis of the cDNA bands and BDD and confirmation of differentially expressed genes revealed discovery of a new gene that is differentially expressed on the experimental group (band 4CC). This gene showed no nucleotide sequence similarities (Fig. 2B). Also, the predicted amino acid sequence was unique.

### Sequencing of the full length gene and protein expression and purification

To obtain the full length cDNA for the selected gene, the full length mRNA was cloned into a mammalian expression vector and the library was screened by specific primers constructed from the original FDD Sequence of band 4CC (Fig. 2B). The full-length sequence was obtained (Fig. 3A) and a geneBank search did not reveal any significant homologies. Open Reading Frames (ORFs) were detected and the first (longest) ORF that was subcloned into bacterial expression vector as a His-Tag Fusion Protein is shown in Fig. 3B. Blat search was carried out in order to map ISRAA sequence to the genome. The search result depicted that ISRAA gene maps to chromosome 14 (chr14:26423008-26425122).

Protein expression was done and rISRAA was purified, characterized and tested for biological activities (see below).

### Biological characterization of rISRAA

Using immunohistochemistry, rISRAA was tested for its ability to produce IFN-γ. Stimulation of mouse splenocytes with rISRAA revealed significant induction of this cytokine (p<0.0001). Co-cultivation of the splenocytes with the anti-rISRAA polyclonal antiserum significantly inhibited the rISRAA-stimulated IFN-γ production (p.0.001). Similarly, the IFN-γ stimulation activity of the 48 hour splenocyte culture supernatants from mice challenged for less than one minute with subcutaneously inoculated *T*.*b*.*brucei* was significantly abrogated by the anti-rISRAA polyclonal antiserum (p.0.001) (Fig. 4A).
SDS electrophoresis and Western blot analysis of rISRAA and the 48 hour splenocyte culture supernatants from mice challenged for less than one minute with subcutaneously inoculated *T*.*b*.*brucei* using the anti-rISRAA polyclonal antiserum showed bands with a molecular weight of ∼15 kD (Fig. 4B and C).

### The role of ISRAA in modulation of innate responses and disease suppression

Immunosuppression is a known feature of late disease of African trypanosomiasis where cells obtained 3 weeks post infections failed to proliferate when stimulated with con-A. However, addition of ISRAA to con-A-stimulated splenocytes obtained at week 3 after infection resulted in a significant proliferative response (p<0.01 when ISRAA added alone and p<0.0001 when ISRAA added with con-A), but no significant proliferative response was recorded with IL-2 compared to non-stimulated cultures (Fig. 5A).

### ISRAA producing and responding cells

To explore the type of cells that can produce ISRAA, mice were challenged for less than one minute with subcutaneously inoculated *T.b.brucei.* Splenocytes were isolated and enriched to obtain purified populations of CD4⁺, CD8⁺, B cells and monocytes/macrophages. All these cell types showed ability to produce ISRAA as detected by immunohistochemistry. In control experiments, splenocytes from non-inoculated mice did not express ISRAA production (Fig. 5B). To further study the type of cells that might responded to ISRAA by producing IFN-γ, mouse splenocytes were stimulated by rISRAA and thereafter CD4⁺, CD8⁺, B cells and monocytes/macrophages were purified and tested for IFN-γ production by immunohistochemistry. As well, all types of cells did respond to ISRAA stimulation since IFN-γ production was registered in all populations. Non-stimulated cells were used as controls and no IFN-γ production was recorded (Fig. 5C).

### Discussion

The data of the present work showed rapid induction of ∼15 kD nervous system-induced Immune System Released Activating Agent (ISRAA) in the spleen within few seconds after parasitic challenge. The appearance of this factor was associated with swift gene expression in the spleen that was shown to be of a unique nucleotide sequence and maps to chromosome 14.

The spleen is richly innervated by adrenergic neurons that enter its parenchyma and remain largely associated with the splenic vasculature. Therefore, the spleen is suggested to play a central role in nervous-to-immune signaling processes documented in this study. In agreement with this suggestion, sympathetic nerve activity was recorded only from the splenic nerve after intravenous injection of LPS into adult rats, with a short onset time of 17.1-23.5 min (MacNeil et al 1996). Interestingly, few seconds' exposure to living parasites resulted in the induction of ISRAA, which activated cells of the immune system to produce IFN-γ and to proliferation. Furthermore, ISRAA is abrogated by sympathetic denervation of the spleen. Less than 1 min time is shorter than the time where electrical activity previously recorded in the splenic nerve (MacNeil et al 1996). However, the challenging agent was different from the very virulent parasite used here, and also electrical activities may start immediately but may not be recorded by our current methods before it is amplified after several minutes. Inhibition of the early innate reactions by splenic denervation downregulated the disease process and prolonged survival of the animals by suppressing parasite growth (Liu et al 2000) and significantly reduced the disease severity in EAMG (Bakhiet et al 2006). Since parasites need their host for survival they may not insult it. However, in their processes to evade the innate immune attack they can harm the host. This can be achieved by for example hiding in the tissue and multiply there or use host derived cytokines (Bakhiet et al 1996), express host homologous molecules (Petry et al 1987, MacNeil et al 1990, Jauberteau 1991, Duvaux-Miret 1992), change their surface coat (Borst 1991) or polyclonally activate the immune cells (Minoprio 1986). One or more of these mechanisms together with overproduction of mediators will certainly negatively affect the state of the immune system and result in immune dysfunction. These mechanisms may explain the mild disease course recorded in the denervated rats (Liu et al 2000, Bakhiet et al 2006) since denervation has abrogated ISRAA and inhibited the early innate immune responses suggesting that inhibition of these reactions especially during early infections may be beneficial for the host.

In contrast to the early effects of ISRAA, this molecule is required for protection from the succeeding immunosuppression that lately evolves during the infection. This was shown by failure of splenocytes to proliferate after con-A-stimulation at week 3 post inoculation. in non-denervated rats, which was ameliorated by denervation or addition of ISRAA, but not IL-2. The consequences of the early immune response may shut off ISRAA in order to slow-down the immune reaction. Whenever ISRAA is required, as in the state of immunosuppression of trypanosomiasis, its gene may already have switched off. This possibly explains the proliferative effects registered after adding ISRAA to the immunosuppressed splenocytes.

In normal life, the host survives many infectious agents in the absence of specific adaptive immunity because of the presence of several protective mechanisms that are independent on specific antigenic recognition. These antigen-independent mechanisms make up the innate immunity. As presented herein, this research has shown that signals generated as a result of contacts between parasites or challenging factors and nerve endings at innate barriers, such as the skin, are transmitted by autonomic (sympathetic or parasympathetic) nerves to the spleen. It has, therefore, been suggested that the spleen accommodates a central control unit (CCU) for the immune system that receives signals during dangerous challenges, such as parasitic infections, and assumes a coordination function in mobilizing defense mechanisms. Without being limited to any particular theory, one current hypothesis is that when the CCU of the spleen receives these alarm signals it gives orders in the form of released mediators such as ISRAA to launch primary responses by cells involved in innate immunity in response to these mediators after the host is challenged with infections. From the relatively low molecular mass of ISRAA, its biological action on broad spectrum of cell types such as T and B cells and monocytes/macrophages and also from the ability of same cell populations to respond to ISRAA by producing cytokines as IFN-γ, it seems that ISRAA is a cytokine of innate immunity. This is supported by the line up of ISRAA to chromosome 14 since several low molecular mass cytokines were mapped to chromosome 14 (Hunt eat 1997). As well, a major locus controlling serum the inflammatory cytokine IL-6 was found on chromosome 14 (Végvári et al 2005).

In conclusion, the present data demonstrates that early communications between the CNS and the immune system during dangerous challenges result in the induction of ISRAA, the first example of a nervous system-induced factor inducing cytokines in innate immune cells. To date there has not been identified any single or group of molecules that function as mediators between the nervous and immune systems in response to a nervous stimulus following an immune challenge. The present work, has, however now identified such a molecule and which molecule may be particularly advantageous in further understanding the mechanism for innate immunity commencement and action. Furthermore, such a mediator also offers particular benefits as a potential therapeutic agent for modulating the natural responses in immune system disorders or in immunosupressed or immunocompromised individuals. In addition, compounds or molecules that inhibit that function of such a mediator may be particularly beneficial as a therapeutic treatment in individuals with an overstimulated immune system.

### References

1. Hadden, J.W., Handden, E.M., and Middleton, E.JR. Lymphocyte blast transformation. Demonstration of adrenergic receptors in human peripheral lymphocyte. Cell. Immunol.1 970; 1: 583-595.
2. Sanders, V.M., and Munson, A.E. Norepine and the antibody response. Phamacolog. Rev. 1985; 37: 229-248.
3. Saito H. Innervation of the guinea pig spleen studied by electron microscopy. Am J Anat. 1990; 189: 213-235.
4. Madden, K.S., and Livnat, S. 1991. Catecholamine action and immunologic reactivity. In Psychoneuroimmunology (ed.), Ader, R., Felten, D.L. and Cohen, N., pp. 283-310. Academic press, N.Y.
5. Elenkov IJ, Wilder RL, Chrousos GP, Vizi ES. The sympathetic nerve--an integrative interface between two supersystems: the brain and the immune system. Pharmacol Rev. 2000; 52: 595-638.
6. Simard, A.R., Rivest, S. Do pathogen exposure and innate immunity cause brain diseases? Neurol Res. 2005; 7: 717-725.
7. Bakhiet, M,, Tjernlund, A., Mousa, A., Gad A., Strömblad, S., Seiger, Å and Andersson, J. Rantes promotes growth and survival of human first trimester forebrain astrocytes. Nature Cell Biol. 2001; 2:150-157.
8. Bakhiet M, Yu L, Ozenci V, Khan A, Shi F-D. Modulation of immune responses and suppression of experimental autoimmune myasthenia gravis by surgical denervation of the spleen. Clin Exp Immunol. 2006; 144: 290-298.
9. Liu Y, Mustafa M, Li H-L, Nuortio L, Mustafa A and Bakhiet M.. Modulation of early immune responses and suppression of Trypanosoma brucei brucei infections by surgical denervation of the spleen. Neuroimmunemodulation. 2000; 8:31-38
10. Liu Y., Li Z., Svaren-Quiding C., Cui J., Ozenci V., and Bakhiet M. 2004. Splenic denervation suppresses mRNA gene expression and protein production of IL-1beta and IL-6 by peritoneal macrophages in both Trypanosoma brucei brucei-infected and non-infected rats. Neuroimmunomodulation. 2004; 11: 113-118.
11. Olsson T., Bakhiet M., Edlund C., Höjeberg B., Van der Meide P.H., and Kristensson, K. 1991. Bidirectional activating signals between Trypanosoma brucei and CD8+ T cells: a trypanosome-released factor triggers interferon-* production that stimulates parasite growth. Eur. J. Immunol. 1991; 21: 2447-2454.
12. Olsson T., Bakhiet M., Höjeberg B., Ljungdahl Å., Edlund C., Andersson G., Ekre H-P, Fung W-P, Leung, Mak T., Wigzell H., Fiszer U., and Kristensson K. 1993. CD8 is critically involved in lymphocyte activation by a Trypanosoma brucei brucei released molecule. Cell 1993; 72: 715-727.
13. Vaidya T., Bakhiet M., Olsson T., Kristensson K., and Donilson J. The gene for a T-lymphocyte triggering factor from African trypanosomes. J. Exp. Med. 1997; 186: 433-438.
14. Askonas BA. Interference in general immune function by parasite infections; African trypanosomiasis as a model system. Parasitology 1984; 88: 633-638.
15. Wei G, Tabel H. Regulatory T cells prevent control of experimental african trypanosomiasis. J Immunol. 2008; 180: 2514-2521.
16. Mustafa, M.I., Diener, P., Höjeberg, B., Van der Meide, P., and Olsson, T. T cell immunity and interferon-γ secretion during experimental allergic encephalomyelitis in Lewis rats. J. Neuroimmunol. 1991; 31:165-177.
17. Van der Meide, P.H., Dubbeld, M., Vijverberg, K., Kos, T., and Schellekens, H. The purification and characterization of rat gamma interferon by use of two monoclonal antibodies. J. Gen. Virol. 1986; 67:1059-1071.
18. Holmdahl, R., Moran, T., and Andersson, M.A. Rapid and efficient immunization protocol for production of monoclonal antibodies reactive with autoantigens. J. Immunol. Methods. 1985; 83: 379-384.
19. Liang P., and Pardee A.B. Differential display of eukaryotic messenger RNA by means of the polymerase chain reaction, Science 1992; 257: 967-971.
20. Sanders, V.M., and Munson, A.E. Norepine and the antibody response. Phamacolog. Rev. 1985; 37:229-248.
21. Lore K, Sonnerborg A, Spetz AL, Andersson U, Andersson J. Immunocytochemical detection of cytokines and chemokines in Langerhans cells and in vitro derived dendritic cells. J Immunol Methods 1998; 214: 97-111.
22. Towbin, H., Staehlin, T. and Gordon J. Electrophoretic transfer of proteins from polyacrylamide gels to nitrocellulose sheets. Proc. Natl. Acad.Sci. 1979; 76: 4350.
23. Mix, E., Olsson, T., Correale, J., Kostulas, V., and Link, H. CD4+, CD8+, and CD4- CD8- T cells in CSF and blood of patients with multiple sclerosis and tension headache. Scand. J. Immunol. 1990; 31, 493-501.
24. MacNeil, B.J., Jansen, A.H., Greenbery, A.H., and Nance, D. M.. Activation and selectivity of splenic sympathetic nerve electrical activity response to bacterial endotoxin. Am. J. Physiol. 1996; 270: 264-270.
25. Bakhiet M., Olsson, T., Mhlanga, J., Büscher, P., Lycke, N., Van der Meide, P., and Kristensson K. Human and rodent IFN-γ as a growth factor for Trypanosoma brucei brucei. Eur. J. Immunol. 1996; 26: 1359-1364.
26. Petry K., Voisin, P., Baltz, T., and Labouesse, J. Epitopes common to trypanosomes (T. cruzi, T. dionisli and T.vespertilionis (Schizotyypcznum): astrocytes and neurons. J. Neuroimmunol. 1987; 16: 237-252.
27. MacNeil I.A., Suda, T., Moore, K.W., Mosmann, T.R., and Zlotnik, A. IL-10, a novel growth cofactor for mature and immature T cells. J. Immunol. 1990; 145: 4167-4173.
28. Jauberteau, M.O., Ben Younes-Chennoufi, A., Amevigbe, M., Bouteille, B., Dumas, M., Breton, J.C., and Baumann, N. Galactocerebrosides are antigens for immunoglobulins in sera of an experimental model of trypanosomiasis in sheep. J. Neurol. Sci. 1991; 101: 82-86.
29. Duvaux-Miret O., Stefano, G.B., Smith, E.M., Dissous, C., and Capron, A. Immunosuppression in the definitive and intermediate hosts of the human parasite Schistosoma mansoni by release of immunoactive neuropeptides. Proc. Natl. Acad. Sci. USA. 1992; 89: 778-781.
30. Borst, P. Molecular genetics of antigenic variation. Immunol Today 1991; 12: 29-33.
31. Minoprio P.M., Eisen, H., Forni, L., D'Imperio Lima, M.R., Joskowicz, M., and Coutinho, A. Polyclonal lymphocyte responses to murine Tryapnosoma cruzi infection. I. Quantitation of both T- and B-cell responses. Scand J Immunol. 1986; 24: 661-668.
32. Hunt JE, Friend DS, Gurish MF, Feyfant E, Sali A, Huang C, Ghildyal N, Stechschulte S, Austen KF, Stevens RL. Mouse mast cell protease 9, a novel member of the chromosome 14 family of serine proteases that is selectively expressed in uterine mast Cells. JBC 1997; 272: 29158-29166.
33. Végvári A, Szabó Z, Szántó S, Nesterovitch AB, Mikecz K, Glant TT, Adarichev VA. Two major interacting chromosome loci control disease susceptibility in murine model of spondyloarthropathy. J Immunol. 2005; 175: 2475-2483.

## Claims

1. An isolated nucleic acid molecule comprising a nucleotide sequence exhibiting at least 70% homology to the sequence represented by the nucleotide sequence of SEQ ID No: 1.

2. An isolated nucleic acid molecule consisting of the nucleic acid sequence according to SEQ ID No:2.

3. An isolated nucleic acid molecule according to any one of claims 1 and 2, wherein said nucleic acid is DNA or RNA, and/or optionally wherein said DNA is a cDNA molecule.

4. An isolated nucleic acid molecule according to any of claims 1 to 3 wherein said isolated nucleic acid molecule is a mammalian nucleic acid molecule.

5. An isolated nucleic acid or oligonucleotide molecule which is antisense to a nucleic acid molecule as claimed in any one of claims 1 to 4.

6. An isolated nucleic acid or oligonucleotide molecule which is antisense to the coding region of a nucleic acid molecule as claimed in any one of claims 1 to 5.

7. An isolated polypeptide molecule encoded by
(i) a nucleic acid molecule according to any of claims 1 to 4; or
(ii) a nucleic acid which hybridises under high stringency conditions to the isolated nucleic acid of any one of claims 1 to 6.

8. An isolated polypeptide molecule having at least 70% homology to the amino acid sequence of SEQ ID NO. 3.

9. An isolated nucleic acid molecule encoding the polypeptide of claim 8.

10. A recombinant expression vector suitable for transformation of a host cell comprising a nucleic acid as claimed in any one of claims 1 to 6 and 9;
and/or optionally wherein the recombinant expression vector is a plasmid;
and/or optionally wherein the recombinant expression vector is a prokaryotic or eukaryotic expression vector; and/or optionally wherein the nucleic acid molecule is operatively linked to a regulatory or expression control sequence;

11. A recombinant expression vector according to claim 10, wherein the nucleic acid is operatively linked to the regulatory or expression control sequence to allow expression of an RNA molecule which is antisense to the isolated nucleic acid of any one of claims 1 to 4, and 9.

12. A transformed host cell comprising the recombinant expression vector of claim 10; and/or optionally wherein the host cell is a eukaryotic or prokaryotic host cell.

13. A method for preparing an isolated ISRAA polypeptide that is induced by a nervous stimulus and initiates immune responses, which method comprises culturing a transformed host cell including a recombinant expression vector as claimed in claim 10 in a suitable medium until ISRAA polypeptide is produced and isolating the resulting polypeptide from said medium.

14. A monoclonal or polyclonal antibody or antigen-binding fragment thereof specific for an epitope of a polypeptide as claimed in any of claims 7 or 8.

15. A pharmaceutical composition comprising a monoclonal or polyclonal antibody as claimed in claim 14 in a pharmaceutically acceptable carrier.

16. A non-human transgenic mammal which contains cells transfected with a nucleic acid molecule according to any one of claims 1 to 6 and 9.

17. The non-human transgenic mammal of claim 16 wherein said mammal is a mouse or a rat.

18. The non-human transgenic mammal of any of claims 16 or 17, comprising a mutation that results from the insertion of a selectable marker gene sequence or other heterologous sequence into the genome of the endognenous *israa* gene by homologous recombination;

19. The non-human transgenic mammal of claim 18 wherein said nucleic acid molecule is expressed in the sense orientation and said mammal expresses a functional ISRAA

20. The non-human transgenic mammal of claim 18 wherein said nucleic acid molecule is expressed in the antisense orientation such that endogenous expression or activity of ISRAA is inhibited at least in the cells of said mammal expressing said nucleic acid.

21. A method for stimulating the innate immune response *in vitro* in immuno-suppressed cells comprising
(i) contacting the cells with purified polypeptide according to any of claim 7 or 8; or
(ii) transfecting the cells with an isolated nucleic acid molecule according to any one of claims 1 to 6 and 9.

22. A method for inhibiting the innate immune response *in vitro* in cells comprising contacting the cells with a polyclonal or monoclonal antibody which binds to a polypeptide according to claim 7 or 8.

23. A method of inhibiting the innate immune response *in vitro* in cells with inflammatory disease comprising inhibiting the activity or expression of a polypeptide according to claim 7 or 8 by:
(i) using a polyclonal or monoclonal antibody specific for said polypeptide; or(ii) wherein the expression of the polypeptide is inhibited by introducing into the cell an isolated nucleic acid molecule or oligonucleotide according to claim 5 or claim 6;
and optionally wherein the isolated nucleic acid molecule or oligonucleotide is RNA, for example, double stranded RNA;
and optionally wherein the isolated nucleic acid molecule is further defined as an oligonucleotide or nucleic acid molecule which is (i) complementary to the sense strand of a genetic sequence that encodes said polypeptide or (ii) to a regulatory region of a gene that encodes the polypeptide or (iii) to a transcription initiation region of a gene that encodes the polypeptide or (iv) to a 5' or 3' untranslated region of a gene which encodes the ISRAA polypeptide or (v) to a translation initiation codon of a gene which encodes said polypeptide;
and optionally wherein the nucleic acid or oligonucleotide molecule is included in a recombinant expression vector which permits expression of said nucleic acid or oligonucleotide in the cell;
and optionally wherein the nucleic acid or oligonucleotide molecule is introduced into cells using at least one delivery vehicle or technique selected from the list comprising viral vectors, microinjection, electroporation, coprecipitation, liposomes and aerosol delivery.

24. A therapeutic kit for inhibiting the innate immune response in cells, said kit comprising a polyclonal or monoclonal antibody which binds to a protein encoded by the nucleic acid of any one of claims 1 to 6 and 9; or to the polypeptide of claim 7 or 8 for incubation with a sample of cells and means of adding the antibody to said cells.

25. A therapeutic kit comprising an isolated nucleic acid molecule or oligonucleotide capable of hybridising to a genetic sequence which encodes the ISRAA polypeptide according to SEQ ID No:3, and means of adding the molecule to cells.

26. A therapeutic kit for stimulating the innate immune response in immuno-suppressed cells comprising
(i) a purified ISRAA protein or a polypeptide according to claim 7 or 8 and means of adding the protein to cells; or
(ii) an isolated nucleic acid according to any one of claims 1 to 6 and 9 and means of adding the isolated nucleic acid to cells.

27. A method of identifying compounds that inhibit or stimulate activity or expression of the polypeptide according to claim 7 or 8 and which method comprises contacting *in vitro* a cell expressing said polypeptide with a candidate compound or agent and establishing whether in response to an immune challenge the compound or agent inhibits or enhances the immune response when compared to a cell that has not been contacted with said compound.

28. The polypeptide according to claim 7 or 8 for use in a method of treating immune system disorders or immunosuppression.

29. The antibody or antigen binding fragment according to claim 14, or the isolated nucleic acid or oligonucleotide molecule according to claim 5 or 6 for use in a method of treating individuals with an overstimulated immune system.

## Patentansprüche

1. Isoliertes Nukleinsäuremolekül, umfassend eine Nukleotidsequenz, die mindestens 70 % Homologie zu der Sequenz aufweist, die dargestellt ist durch die Nukleotidsequenz von SEQ ID NR.: 1.

2. Isoliertes Nukleinsäuremolekül, bestehend aus der Nukleinsäuresequenz nach SEQ ID NR.:2.

3. Isoliertes Nukleinsäuremolekül nach einem der Ansprüche 1 und 2, wobei die Nukleinsäure DNA oder RNA ist und/oder wobei die DNA optional ein cDNA-Molekül ist.

4. Isoliertes Nukleinsäuremolekül nach einem der Ansprüche 1 bis 3, wobei das isolierte Nukleinsäuremolekül ein Säugetier-Nukleinsäuremolekül ist.

5. Isoliertes Nukleinsäure- oder Oligonukleotidmolekül, das antisense zu einem Nukleinsäuremolekül nach einem der Ansprüche 1 bis 4 ist.

6. Isoliertes Nukleinsäure- oder Oligonukleotidmolekül, das antisense zu der Kodierregion eines Nukleinsäuremoleküls nach einem der Ansprüche 1 bis 5 ist.

7. Isoliertes Polypeptidmolekül, kodiert durch
(i) ein Nukleinsäuremolekül nach einem der Ansprüche 1 bis 4; oder
(ii) eine Nukleinsäure, die unter hochstringenten Bedingungen an die isolierte Nukleinsäure nach einem der Ansprüche 1 bis 6 hybridisiert.

8. Isoliertes Polypeptidmolekül, das mindestens 70 % Homologie aufweist zu der Aminosäuresequenz von SEQ ID NR. 3.

9. Isoliertes Nukleinsäuremolekül, das für das Polypeptid nach Anspruch 8 kodiert.

10. Rekombinanter Expressionsvektor, der zum Transformieren einer Wirtszelle geeignet ist, die eine Nukleinsäure nach einem der Ansprüche 1 bis 6 und 9 umfasst;
und/oder wobei der rekombinante Expressionsvektor optional ein Plasmid ist;
und/oder wobei der rekombinante Expressionsvektor optional ein prokaryotischer oder eukaryotischer Expressionsvektor ist; und/oder wobei das Nukleinsäuremolekül optional operativ an eine Regulator- oder Expressionskontrollsequenz gebunden ist.

11. Rekombinanter Expressionsvektor nach Anspruch 10, wobei die Nukleinsäure operativ an die Regulator- oder Expressionskontrollsequenz gebunden ist, um eine Expression eines RNA-Moleküls zu ermöglichen, das antisense zu der isolierten Nukleinsäure nach einem der Ansprüche 1 bis 4 und 9 ist.

12. Transformierte Wirtszelle, umfassend den rekombinanten Expressionsvektor nach Anspruch 10; und/oder wobei die Wirtszelle optional eine eukaryotische oder prokaryotische Wirtszelle ist.

13. Verfahren zur Herstellung eines isolierten ISRAA-Polypeptids, das durch einen Nervenreiz induziert ist und Immunantworten initiiert, wobei das Verfahren das Kultivieren einer transformierten Wirtszelle einschließlich eines rekombinanten Expressionsvektors nach Anspruch 10 in einem geeigneten Medium, bis ein ISRAA-Polypeptid hergestellt ist, und das Isolieren des resultierenden Polypeptids aus dem Medium umfasst.

14. Monoklonaler oder polyklonaler Antikörper oder Antigen-bindendes Fragment davon, spezifisch für ein Epitop eines Polypeptids nach einem der Ansprüche 7 oder 8.

15. Pharmazeutische Zusammensetzung, umfassend einen monoklonalen oder polyklonalen Antikörper nach Anspruch 14 in einem pharmazeutisch unbedenklichen Träger.

16. Nicht menschliches transgenes Säugetier, das Zellen enthält, die mit einem Nukleinsäuremolekül nach einem der Ansprüche 1 bis 6 und 9 transfiziert sind.

17. Nicht menschliches transgenes Säugetier nach Anspruch 16, wobei das Säugetier eine Maus oder Ratte ist.

18. Nicht menschliches transgenes Säugetier nach einem der Ansprüche 16 oder 17, umfassend eine Mutation, die aus dem Einführen einer auswählbaren Markergensequenz oder anderen heterologen Sequenz in das Genom des endogenen *israa*-Gens durch homologe Rekombination resultiert.

19. Nicht menschliches transgenes Säugetier nach Anspruch 18, wobei das Nukleinsäuremolekül in der Sense-Richtung exprimiert ist und das Säugetier einen funktionalen ISRAA exprimiert.

20. Nicht menschliches transgenes Säugetier nach Anspruch 18, wobei das Nukleinsäuremolekül in der Antisense-Richtung exprimiert ist, sodass endogene Expression oder Aktivität von ISRAA mindestens in den Zellen des Säugetiers gehemmt wird, die die Nukleinsäure exprimieren.

21. Verfahren zum Stimulieren der angeborenen Immunantwort *in vitro* in immunsupprimierten Zellen, umfassend
(i) das Kontaktieren der Zellen mit gereinigtem Polypeptid nach einem der Ansprüche 7 oder 8; oder
(ii) das Transfizieren der Zellen mit einem isolierten Nukleinsäuremolekül nach einem der Ansprüche 1 bis 6 und 9.

22. Verfahren zum Hemmen der angeborenen Immunantwort *in vitro* in Zellen, umfassend das Kontaktieren der Zellen mit einem polyklonalen oder monoklonalen Antikörper, der an ein Polypeptid nach Anspruch 7 oder 8 bindet.

23. Verfahren zum Hemmen der angeborenen Immunantwort *in vitro* in Zellen mit Entzündungskrankheit, umfassend das Hemmen der Aktivität oder Expression eines Polypeptids nach Anspruch 7 oder 8 durch:
(i) Verwenden eines polyklonalen oder monoklonalen Antikörpers, der spezifisch für das Polypeptid ist; oder (ii) wobei die Expression des Polypeptids durch Einführen eines isolierten Nukleinsäuremoleküls oder Oligonukleotids nach Anspruch 5 oder Anspruch 6 in die Zelle gehemmt wird;
und wobei das isolierte Nukleinsäuremolekül oder Oligonukleotid optional RNA ist, zum Beispiel doppelsträngige RNA;
und wobei das isolierte Nukleinsäuremolekül optional ferner als Oligonukleotid- oder Nukleinsäuremolekül definiert ist, das (i) komplementär zu dem Sense-Strang einer genetischen Sequenz ist, die für das Polypeptid kodiert, oder (ii) zu einer Regulatorregion eines Gens, das für das Polypeptid kodiert, oder (iii) zu einer Transkriptionsinitiationsregion eines Gens, das für das Polypeptid kodiert, oder (iv) zu einer 5' oder 3' untranslatierten Region eines Gens, das für das ISRAA-Polypeptid kodiert, oder (v) zu einem Translationsinitiationscodon eines Gens, das für das Polypeptid kodiert;
und wobei das Nukleinsäure- oder Oligonukleotidmolekül optional in einen rekombinanten Expressionsvektor eingeschlossen ist, der die Expression der Nukleinsäure oder des Oligonukleotids in der Zelle ermöglicht;
und wobei das Nukleinsäure- oder Oligonukleotidmolekül optional unter Verwendung von mindestens einem Abgabevehikel oder einer Technik ausgewählt aus der Liste umfassend virale Vektoren, Mikroinjektion, Elektroporation, Mitfällung, Liposome und Aerosolabgabe in Zellen eingeführt wird.

24. Therapeutisches Kit zum Hemmen der angeborenen Immunantwort in Zellen, wobei das Kit einen polyklonalen oder monoklonalen Antikörper, der an ein Protein bindet, das durch die Nukleinsäure nach einem der Ansprüche 1 bis 6 und 9 kodiert ist; oder an das Polypeptid nach Anspruch 7 oder 8 zur Inkubation mit einer Probe von Zellen und Mittel zum Hinzufügen des Antikörpers zu den Zellen umfasst.

25. Therapeutisches Kit, umfassend ein isoliertes Nukleinsäuremolekül oder Oligonukleotid, das dazu in der Lage ist, an eine genetische Sequenz zu hybridisieren, die für das ISRAA-Polypeptid nach SEQ ID NR.:3 kodiert, und Mittel zum Hinzufügen des Moleküls zu Zellen.

26. Therapeutisches Kit zum Stimulieren der angeborenen Immunantwort in immunsupprimierten Zellen, umfassend
(i) ein gereinigtes ISRAA-Protein oder ein Polypeptid nach Anspruch 7 oder 8 und Mittel zum Hinzufügen des Proteins zu Zellen; oder
(ii) eine isolierte Nukleinsäure nach einem der Ansprüche 1 bis 6 und 9 und Mittel zum Hinzufügen der isolierten Nukleinsäure zu Zellen.

27. Verfahren zum Identifizieren von Verbindungen, die Aktivität oder Expression des Polypeptids nach Anspruch 7 oder 8 hemmen oder stimulieren, und wobei das Verfahren das Kontaktieren einer Zelle, die das Polypeptid exprimiert, *in vitro* mit einer Kandidatenverbindung oder einem Wirkstoff umfasst und das Feststellen, ob die Verbindung oder der Wirkstoff in Antwort auf eine Immunprovokation die Immunantwort im Vergleich zu einer nicht mit der Verbindung kontaktierten Zelle hemmt oder verbessert.

28. Polypeptid nach Anspruch 7 oder 8 zur Verwendung in einem Verfahren zur Behandlung von Störungen des Immunsystems oder Immunsuppression.

29. Antikörper oder Antigen-bindendes Fragment nach Anspruch 14 oder isoliertes Nukleinsäure- oder Oligonukleotidmolekül nach Anspruch 5 oder 6 zur Verwendung in einem Verfahren zur Behandlung von Individuen mit einem überstimulierten Immunsystem.

## Revendications

1. Molécule d'acide nucléique isolée comprenant une séquence de nucléotides présentant au moins 70% d'homologie avec la séquence représentée par la séquence des nucléotides de séquence SEQ ID NO: 1.

2. Molécule d'acide nucléique isolée composée de la séquence d'acides nucléiques selon la séquence SEQ ID NO: 2.

3. Molécule d'acide nucléique isolée selon l'une quelconque des revendications 1 et 2, où ledit acide nucléique est l'ADN ou l'ARN, et/ou éventuellement où ledit l'ADN est une molécule d'ADNc.

4. Molécule d'acide nucléique isolée selon l'une quelconque des revendications 1 à 3, où ladite molécule d'acide nucléique isolée est une molécule d'acide nucléique de mammifères.

5. Molécule d'oligonucléotide ou d'acide nucléique isolé, qui est antisens à une molécule d'acide nucléique selon l'une quelconque des revendications 1 à 4.

6. Molécule d'oligonucléotide ou d'acide nucléique isolé, qui est antisens à la zone de codage d'une molécule d'acide nucléique selon l'une quelconque des revendications 1 à 5.

7. Molécule de polypeptide isolée codée par
(i) une molécule d'acide nucléique selon l'une quelconque des revendications 1 à 4 ; ou
(ii) un acide nucléique qui s'hybride dans des conditions très rigoureuses avec l'acide nucléique isolé selon l'une quelconque des revendications 1 à 6.

8. Molécule de polypeptide isolée ayant au moins 70% d'homologie avec la séquence des acides aminés de séquence SEQ ID NO: 3.

9. Molécule d'acide nucléique isolée selon la revendication 8.

10. Vecteur d'expression recombinant convenant à la transformation d'une cellule hôte comprenant un d'acide nucléique selon l'une quelconque des revendications 1 à 6 et 9 ; et/ou éventuellement où le vecteur d'expression recombinant est un plasmide ; et/ou éventuellement où le vecteur d'expression recombinant est un vecteur d'expression procaryote ou eucaryote ; et/ou éventuellement où la molécule d'acide nucléique est liée de manière opérationnelle à une séquence de contrôle d'expression ou de régulation.

11. Vecteur d'expression recombinant selon la revendication 10, où l'acide nucléique est lié de manière opérationnelle à la séquence de contrôle d'expression ou de régulation afin de permettre l'expression d'une molécule d'ARN qui est antisens de l'acide nucléique isolé selon l'une quelconque des revendications 1 à 4 et 9.

12. Cellule hôte transformée comprenant le vecteur d'expression recombinant selon la réclamation 10 ; et/ou éventuellement où la cellule hôte est une cellule hôte eucaryote ou procaryote.

13. Procédé de préparation d'un polypeptide ISRAA isolé, préparation induite par une stimulation nerveuse et initiant des réponses immunitaires, lequel procédé comprend la culture d'une cellule hôte transformée contenant un vecteur d'expression recombinant selon la revendication 10 dans un milieu approprié jusqu'à la production de polypeptide ISRAA, et l'isolation du polypeptide résultant à partir dudit milieu.

14. Anticorps monoclonal ou polyclonal ou fragment de liaison d'antigène de celui-ci pour un épitope de polypeptide selon l'une quelconque des revendications 7 ou 8.

15. Composition pharmaceutique comprenant un anticorps monoclonal ou polyclonal selon la revendication 14 dans un véhicule acceptable du point de vue pharmaceutique.

16. Mammifère transgénique non humain qui contient des cellules transfectées avec une molécule d'acide nucléique selon l'une quelconque des revendications 1 à 6 et 9.

17. Mammifère transgénique non humain selon la revendication 16, où ledit mammifère est une souris ou un rat.

18. Mammifère transgénique non humain selon l'une quelconque des revendications 16 ou 17, comprenant une mutation qui découle de l'insertion d'une séquence de gène marqueur sélectionnable ou autre séquence hétérologue dans le génome du gène *israa* endogène par recombinaison homologue.

19. Mammifère transgénique non humain selon la revendication 18, où ladite molécule d'acide nucléique est exprimée dans l'orientation sens et ledit mammifère exprime une ISRAA fonctionnelle.

20. Mammifère transgénique non humain selon la revendication 18, où ladite molécule d'acide nucléique est exprimée dans l'orientation antisens pour inhiber cette expression ou activité endogène de l'ISRAA au moins dans les cellules dudit mammifère exprimant ledit acide nucléique.

21. Procédé permettant de stimuler la réponse immunitaire innée *in vitro* dans les cellules immunodéprimées comprenant
(i) la mise en contact des cellules avec du polypeptide purifié selon l'une quelconque des revendications 7 ou 8 ; ou
(ii) la transfection des cellules avec une molécule d'acide nucléique isolée selon l'une quelconque des revendications 1 à 6 et 9.

22. Procédé permettant d'inhiber la réponse immunitaire innée *in vitro* dans les cellules comprenant la mise en contact des cellules avec un anticorps polyclonal ou monoclonal qui se lie à un polypeptide selon les revendications 7 ou 8.

23. Procédé permettant d'inhiber la réponse immunitaire innée *in vitro* dans les cellules présentant une maladie inflammatoire comprenant l'inhibition de l'activité ou de l'expression d'un polypeptide selon les revendications 7 ou 8 en :
(i) utilisant un anticorps polyclonal ou monoclonal spécifique pour ledit polypeptide ; Ou
(ii) où l'expression du polypeptide est inhibée en introduisant dans la cellule une molécule d'acide nucléique isolé ou un oligonucléotide selon la revendication 5 ou la revendication 6 ; ;
et éventuellement où la molécule d'acide nucléique isolé ou l'oligonucléotide est un ARN, par exemple un ARN double brin ;
et éventuellement où la molécule d'acide nucléique isolée est définie en outre comme une molécule d'oligonucléotide ou d'acide nucléique qui est (i) complémentaire du brin sens d'une séquence génétique codante pour ledit polypeptide ou (ii) d'une zone de régulation d'un gène qui encode le polypeptide ou (iii) d'une zone d'initiation de transcription d'un gène qui encode le polypeptide ou (iv) d'une zone non traduite 5' ou 3' d'un gène qui encode le polypeptide ISRAA ou (v) d'un codon d'initiation de traduction d'un gène qui encode ledit polypeptide ;
et, éventuellement où la molécule d'acide nucléique ou d'oligonucléotide est incluse dans un vecteur d'expression recombinant qui permet l'expression dudit acide nucléique ou dudit oligonucléotide dans la cellule ;
et, éventuellement où la molécule d'acide nucléique ou d'oligonucléotide est introduit dans des cellules à l'aide d'au moins un véhicule d'administration ou d'au moins une technique d'administration sélectionnés dans la liste comprenant les vecteurs viraux, la microinjection, l'électroporation, la coprécipitation, les liposomes et l'administration par aérosol.

24. Kit thérapeutique pour inhiber la réponse immunitaire innée chez les cellules, ledit kit comprenant un anticorps polyclonal ou monoclonal qui se lie à une protéine codée par l'acide nucléique selon l'une quelconque des revendications 1 à 6 et 9 ; ou au polypeptide selon les revendications 7 ou 8 pour incubation avec un échantillon de cellules et des moyens pour ajouter l'anticorps auxdites cellules.

25. Kit thérapeutique comprenant une molécule d'acide nucléique isolée ou un oligonucléotide capables de s'hybrider avec une séquence génétique qui encode le polypeptide ISRAA selon la séquence SEQ ID NO:3, et des moyens pour ajouter la molécule aux cellules.

26. Kit thérapeutique permettant de stimuler la réponse immunitaire innée in vitro dans les cellules immunodéprimées comprenant
(i) une protéine ISRAA purifiée ou un polypeptide selon les revendications 7 ou 8 et des moyens pour ajouter la protéine aux cellules ; ou
(ii) un acide nucléique isolé selon l'une quelconque des revendications 1 à 6 et 9 et des moyens pour ajouter l'acide nucléique isolé aux cellules.

27. Procédé pour identifier des composés qui inhibent ou stimulent l'activité ou l'expression du polypeptide selon les revendications 7 ou 8 et lequel procédé comprend la mise en contact *in vitro* d'une cellule exprimant ledit polypeptide avec un composé candidat ou l'agent et consiste à décider si, en réponse à un défi immunitaire le composé ou l'agent inhibe ou améliore la réponse immunitaire par rapport à une cellule qui n'a pas été mise en contact avec ledit composé.

28. Polypeptide selon les revendications 7 ou 8 pour utilisation dans un procédé de traitement d'affections du système immunitaire ou d'une immunosuppression.

29. Anticorps ou fragment de liaison d'antigène selon la revendication 14, ou molécule d'acide nucléique isolé ou d'oligonucléotide selon les revendications 5 ou 6 pour utilisation dans un procédé de traitement de personnes au système immunitaire hyperstimulé.


### SEQUENCE LISTING

<110> Arabian Gulf University Bakhiet, Abdelmoiz
<120> Immune System Mediator
<130> 1154-0002
<150> GB0703887.0
   <151> 2007-02-28
<160> 3
<170> PatentIn version 3.3
<210> 1
   <211> 2093
   <212> DNA
   <213> Rattus sp.
<400> 1
   cccagttagt gcctgagtac gagagactca tcagggctga gaaaggagaa tggcagggga     60
   aacagtgcta caaggatgtc ctttgtgtcc tgaccatgca gaaggagaca gaagccccag    120
   gattggtggt tcccagaggc agagcccact cctggaggtg actcacactc agtgttgtca    180
   tttgatcctt agctgtctgt atgtctggtg ccctgtgacc cagtctcttt atggtgctcc    240
   ggagcagcat tcctttctgt ccccccttgt tcttctccac gcttcccaac gaccaggtac    300
   acacccactt gccatctccc tgttaaccct accttcccag ttagtgcctg agtacacaga    360
   agtgacatct ctccaggtca tcagaattct tgtcaatccc tctgagtcag caaactgcct    420
   gggttagaag tctgggactt ctctgggact cagaacccaa tgcccagaat ctgagccaca    480
   tcaaggctgg ttgttctccc tctcctcatc ttgagagttt ggcttgtgca tgacaggcct    540
   gcctgcccac ttagttagcc ttccttcctt tcttcctaac ttcctgatgg ccttcgaagc    600
   ccctagactt accatatcac taccaaaaga aaaaagaaaa agaaaaagct gtaagtggga    660
   agtgacccac ttttgacccc tggtacatgc atcccaggta gatgccttgg tcccttgtct    720
   tattggaact gattctagct catccatgag ctgaaggaag tcagtccttt gacccaagct    780
   tgggactgtg gtgtcctggc tacattatct cccttctcac ccctcctcta gagggtcctt    840
   cagggggcgg gctccccact gccagaggat aaagcatccc cgactccgga gccagccagc    900
   catgccacag cactgtattt attggcggct cctcttcact caggcccacg acaggagttc    960
   agacccccgc gagaaaagcc acccgctatt gatccttagt caggtctccg gagcctggca   1020
   gacattcatc aaggagccac agcatggggc tacagctcgc cctttctttc ttcttccttt   1080
   cttctacccc ccaagtcatt cccccccccc gggggggggt ccgagggagg cgggtgaggg   1140
   ggcttgccga ggtcactcca agcactgcgg ggtggggtgg ggtggggtgg ggttggctgt   1200
   gggccccggg cttttgagca agtctttgga agttagagat gtggaacttg gagcgagtca   1260
   gggaaagccc ttctaacgaa gatagtgtgt ggagtgccgg catttctgta gactccaata   1320
   ctaaaaagag gctgctaacc aagagggcct tgagggattt gattccagag aagagagcct   1380
   agaaattagc aagtgaaagg ggccagtttg gactgatgaa tgggtgcttg aaaacaattt   1440
   gttttcttaa gtatgtaaat atgctagtga agagtattta ctttttgggt taccttggaa   1500
   aaccctgtat cttgaatatt atttccatcc tcagggtccc actcaggagc ttctgaagtg   1560
   ggtcccgagg agaactaaga ggtaggagga cgaggaggca aaacaacggg aagctgtctt   1620
   tataattttt aaaggattac caccaccaag tccctgcccc taaggtgtaa actttgctca   1680
   gttccagcct gaatgttcac tcaacctgca ggaatgatct tttttaatat atgtatttca   1740
   aaatatatgt atattttatt tatatatgta tatatcatgt atgaatatgt ttgcttgctt   1800
   atatgtccgt gttccacttg tgtgtttggt gccctcggaa tagaagaggg tgttgaattt   1860
   ccctaaacct ggagtttcag atgattgtga gctgccacat gggtgccaag gagagaattt   1920
   tggccctttg caagagcaag aaaccttttt taaccactga gccatttttc ctgccccagg   1980
   actttttttg taaagttagt tctttttgct actgtgtggg taagcccttg gccactgaac   2040
   aggccctatg aatcagaaaa aaaaaaaaaa aaaaaaaaaa aaaaaaaaaa aaa          2093
<210> 2
   <211> 378
   <212> DNA
   <213> Rattus sp.
<400> 2
   atggcagggg aaacagtgct acaaggatgt cctttgtgtc ctgaccatgc agaaggagac      60
   agaagcccca ggattggtgg ttcccagagg cagagcccac tcctggaggt gactcacact     120
   cagtgttgtc atttgatcct tagctgtctg tatgtctggt gccctgtgac ccagtctctt     180
   tatggtgctc cggagcagca ttcctttctg tccccccttg ttcttctcca cgcttcccaa      240
   cgaccaggta cacacccact tgccatctcc ctgttaaccc taccttccca gttagtgcct     300
   gagtacacag aagtgacatc tctccaggtc atcagaattc ttgtcaatcc ctctgagtca     360
   gcaaactgcc tgggttag                                                   378
<210> 3
   <211> 125
   <212> PRT
   <213> Rattus sp.
<400> 3
   Met Ala Gly Glu Thr Val Leu Gln Gly Cys Pro Leu Cys Pro Asp His
   1               5                   10                  15
   Ala Glu Gly Asp Arg Ser Pro Arg Ile Gly Gly Ser Gln Arg Gln Ser
               20                  25                  30
   Pro Leu Leu Glu Val Thr His Thr Gln Cys Cys His Leu Ile Leu Ser
           35                  40                  45
   Cys Leu Tyr Val Trp Cys Pro Val Thr Gln Ser Leu Tyr Gly Ala Pro
       50                  55                  60
   Glu Gln His Ser Phe Leu Ser Pro Leu Val Leu Leu His Ala Ser Gln
   65                  70                  75                  80
   Arg Pro Gly Thr His Pro Leu Ala Ile Ser Leu Leu Thr Leu Pro Ser
                   85                  90                  95
   Gln Leu Val Pro Glu Tyr Thr Glu Val Thr Ser Leu Gln Val Ile Arg
               100                 105                 110
   Ile Leu Val Asn Pro Ser Glu Ser Ala Asn Cys Leu Gly
           115                 120                 125